# FASCICULE DE BREVET EUROPEEN

(11) **EP 3 317 326 B1**
(45) Date de publication et mention de la délivrance du brevet: **18.11.2020**
(21) Numéro de dépôt: 16750922.3
(22) Date de dépôt: 01.07.2016
(51) Int. Cl.: C08J 3/05, C08J 3/075

(54) **PROCÉDÉ DE FABRICATION D'HYDROGEL À BASE DE CHITOSAN ET DE POLYÉLECTROLYTES CHARGÉS NÉGATIVEMENT ET MATÉRIAU POREUX ALVÉOLAIRE ISSU DUDIT HYDROGEL**
VERFAHREN ZUR HERSTELLUNG VON HYDROGEL MIT CHITOSAN UND NEGATIV GELADENE POLYELEKTROLYTEN UND ZELLULÄRES, PORÖSES MATERIAL AUS DIESEM HYDROGEL
METHOD FOR THE PRODUCTION OF HYDROGEL COMPRISING CHITOSAN AND NEGATIVELY CHARGED POLYELECTROLYTES, AND CELLULAR, POROUS MATERIAL RESULTING FROM SAID HYDROGEL

(30) Priorité: 02.07.2015 FR 1556283
(43) Date de publication de la demande: 09.05.2018
(73) Titulaire: Université de Lille, 59800 Lille (FR); Centre National de la Recherche Scientifique, 75794 Paris Cedex 16 (FR); Institut National de la Santé et de la Recherche Médicale (INSERM), 75013 Paris (FR); Centre Hospitalier Régional Universitaire de Lille, 59037 Lille Cedex (FR)
(72) Inventeur: BLANCHEMAIN, Nicolas, 59660 Merville (FR); MARTEL, Bernard, 59850 Nieppe (FR); FLORES, Claudia, 59000 Lille (FR); CAZAUX, Frédéric, 59870 Marchiennes (FR); CHAI, Feng, 59120 Loos (FR); TABARY, Nicolas, 59800 Lille (FR); LOPEZ HEREDIA, Marco, 59300 Valenciennes (FR)
(74) Mandataire: Cabinet Beau de Loménie
(86) Numéro de dépôt international: PCT/FR2016/051684
(87) Numéro de publication internationale: WO 2017/001808

(56) Documents cités:
- WO-A1-99/07416
- WO-A1-2013/015688
- WO-A1-2015/120085
- WO-A2-2011/070529
- FR-A1- 2 903 594
- US-A- 6 039 470
- US-A1- 2008 254 094
- EMILIE PATOIS ET AL: "Novel thermosensitive chitosan hydrogels: In vivo evaluation", JOURNAL OF BIOMEDICAL MATERIALS RESEARCH. PART A, vol. 91A, no. 2, 1 novembre 2009 (2009-11-01), pages 324-330, XP055220738, HOBOKEN, NY, US ISSN: 1549-3296, DOI: 10.1002/jbm.a.32211

## Description

La présente invention concerne le domaine technique des procédés de fabrication des hydrogels, notamment implantables, ainsi que le domaine technique des procédés de fabrication des matériaux alvéolaires obtenus par l'intermédiaire desdits hydrogels, en particulier concernant le domaine technique des procédés de fabrication des hydrogels physiques.

La présente invention concerne également le domaine technique des hydrogels, des matériaux alvéolaires (notamment des éponges) issus desdits hydrogels, et des dispositifs médicaux comprenant de tels hydrogels ou matériaux alvéolaires.

### Arrière-plan de l'invention

Les hydrogels ou gels macromoléculaires comprenant très majoritairement de l'eau, sont des matrices de polymère(s) gonflées par une grande quantité d'eau. Les macromolécules sont interconnectées entre elles, formant un réseau constituant un échafaudage (scaffold) qui confère au gel les propriétés d'un matériau aux propriétés viscoélastiques.

Les liaisons entre chaînes polymères peuvent être de deux formes : permanentes ou réversibles. Quand elles sont permanentes, on observe la présence de liaisons covalentes, on parle alors d'hydrogels chimiques. Lorsque les liaisons entre les chaînes sont réversibles, les macromolécules interagissent via des liaisons non covalentes de types hydrogènes, ioniques ou hydrophobes, dipôle-dipôle on parle alors d'hydrogels physiques.

Les hydrogels peuvent être utilisés en tant que biomatériaux, c'est-à-dire en tant que dispositifs médicaux, notamment implantables. De tels hydrogels peuvent comprendre un réseau polymère à base de polysaccharides naturels (par exemple : l'amidon, la cellulose, les carraghénanes, les alginates...), de protéines (gélatine), ou à base de polymères synthétiques tels que les polyacrylamides, les polyacrylates (« carbomères »), formant des réseaux macromoléculaires tridimensionnels enchevêtrés et stabilisés par des liaisons covalentes (gels chimiques irréversibles ou permanents) ou par des liaisons hydrogènes, hydrophobes, dipolaires (dipôle-dipôle) ou encore électrostatiques (gels physiques réversibles).

Le chitosan (CHT), appartenant à la famille de polysaccharides disponibles naturellement, est le seul polymère cationique d'origine naturelle connu à ce jour (polyélectrolyte chargé positivement, désigné sous la référence PE⁺), il est produit par désacétylation de la chitine, ou extrait directement des champignons. Le chitosan est un copolymère composé de la distribution aléatoire de D-glucosamine liée en β-1,4 et de N-acétyl-D-glucosamine. On caractérise un chitosan par son degré de désacétylation, qui représente le pourcentage d'unités de répétitions glucosamine sur sa chaîne macromoléculaire.

La chitine est le composant de l'exosquelette des arthropodes (crustacés) ou de l'endosquelette des céphalopodes. Le chitosan se caractérise par sa forte teneur en fonctions amines (de l'ordre de 5 millimoles (mmol) par gramme, selon son degré de désacétylation (couramment compris entre 60% et 90%). Le CHT est très utilisé dans des applications biomédicales pour ses propriétés de biocompatibilité, de bio-résorbabilité, d'hémocompatibilité, cicatrisantes, et pour son action contre le développement de souches bactériennes.

Le chitosan est un polymère disponible sous forme de poudre, ou de flocons de granulométrie plus ou moins élevée. Il est le seul polymère aminé naturel à se présenter sous la forme d'une poudre, insoluble dans l'eau pure, mais soluble dès acidification de l'eau par au minimum 1% (vol/vol) d'acide acétique concentré. D'autres polyamines portant des fonctions amines primaires (-NH₂) existent, telles que la polyvinylamine, la polyallylamine et la polyéthylèneimine mais elles sont seulement disponibles à l'état de solutions aqueuses concentrées, ou solides sous la forme d'une gomme (car très hygroscopiques) et ne constituent pas des poudres présentant de la fluidité ou un libre écoulement.

La réticulation du CHT par voie ionique pour l'obtention d'un hydrogel est une voie douce et simple à réaliser car elle se produit spontanément, sans chauffage ni catalyseur. On utilise ainsi des agents réticulants anioniques avec des sulfates, des citrates, des phosphates ou des polyphosphates, des polyoxyanions, ou des sels de calcium. La méthode consiste à laisser tremper l'« objet » en CHT (film, monolythe etc.) dans une solution aqueuse contenant l'agent réticulant qui diffuse à l'intérieur de l'« objet » en même temps que l'eau qui provoque son gonflement et la formation d'un gel. Les procédés alternatifs consistent à ajouter l'agent réticulant dans la solution aqueuse de chitosan préalablement dissout dans une solution diluée d'acide acétique (souvent à au moins 0.5% d'acide acétique par exemple), ou à l'inverse, la solution de CHT est ajoutée goutte à goutte dans la solution d'agent réticulant, au moyen d'une seringue par exemple (J. Berger et al./European Journal of Pharmaceutics and Biopharmaceutics 57 (2004) 19-34).

Les cyclodextrines, appartenant également à la famille des polysaccharides, sont des molécules cages capables de former des complexes d'inclusion réversibles avec de nombreux principes actifs notamment lipophiles. Les cyclodextrines sont des polysaccharides cycliques issus d'une dégradation enzymatique de l'amidon. Elles sont constituées d'un enchainement de six à huit unités gluco-pyranoses, reliées entre elles par des liaisons a-(1,4) formant un macrocycle dont le pourtour est riche en fonctions hydroxyles qui apportent la solubilité dans l'eau, tandis que l'intérieur forme une cavité apolaire et hydrophobe conférant aux cyclodextrines des propriétés de complexation avec des principes actifs lipophiles par formation de complexes d'inclusion. On connait des gels chimiques (non réversibles) à base de cyclodextrines obtenus par réticulation d'une cyclodextrine avec l'épichlorhydrine, un diépoxyde, un diisocyanate, le dianhydride pyromellitique, le trimetaphosphate de sodium ou l'EDTA utilisés en tant qu'agents de réticulation. Ces polymères de cyclodextrines réticulés ou « hyperbranchés » forment soit des gels macroscopiques insolubles, ou des nanogels solubles qui présentent des propriétés de piégeage et de vectorisation de molécules organiques, dont des agents thérapeutiques (Progress in Polymer Science, Volume 38, Issue 2, February 2013, Pages 344-368, Advanced Drug Delivery Review 65 (2013) 1188-1203 ; Belstein Journal of Organic Chemistry, 2014, 10, 2586-2593).

Des travaux antérieurs combinant la cyclodextrine et le chitosan ont été publiés. Toutefois ces travaux n'ont pas consisté à obtenir un gel physique macroscopique à partir du mélange d'un polymère de cyclodextrine, et de chitosan. Par exemple, Auzely (C.R. Chimie 14 (2011) 167-177) a réalisé un greffage covalent de cyclodextrine (CD) sur le chitosan (= CHT-CD), d'une part, et le greffage d'adamantane (ada) sur le chitosan (= CHT-ada) d'autre part. Ces deux polymères une fois mélangés en solution forment un gel macroscopique supramoléculaire par formation d'un réseau formé de ces deux types de polymères, provoqué par la formation spontanée de complexes d'inclusion entre les cyclodextrines et l'adamantane, formant un hydrogel stabilisé par des interactions de type supramoléculaires entre les réseaux interpénétrés de CHT-ada et de CHT-CD. De plus la très forte stabilité du complexe CD-ada ne permet pas dans les mélanges l'inclusion d'une molécule tierce, tel qu'un agent thérapeutique dans les cavités des cyclodextrines si la stoechiométrie (ou le rapport molaire) CD/ada est inférieure à 1, toutes les cavités étant irréversiblement complexées par un groupe adamantane.

La plupart des gels commercialisés comme dispositifs médicaux sont prêts à l'emploi, c'est-à-dire déjà hydratés, et contenant une charge minérale, par exemple le phosphate de calcium ou le béta triphosphate de calcium, ou encore une molécule thérapeutique active. On peut citer l'In'oss™, qui est un gel injectable à base de HPMC (hydroxypropylmethylcellulose) contenant de l'hydroxyapatite pour le comblement osseux ; le Periochip® ou Atridox® à base de gélatine et de polymère de PLA (acide polylactique) respectivement contenant un principe actif pour une application en parodontologie. Il existe également des gels pour le comblement des rides à base d'acide hyaluronique ou de collagène, ou encore des enductions de gels sur un panneau textile à base de CMC (carboxyméthylcellulose), éventuellement chargés en un principe actif, servant à la confection des pansements ayant pour fonction de maintenir l'hydratation de la plaie ou de libérer un principe actif. La société Biom'Up commercialise un matériau de comblement osseux (Matribone ®) sous la forme d'une éponge à base de collagène incluant une charge de poudre de bétatriphosphate de calcium.

Il existe également des hydrogels physiques formés d'un complexe ionique résultant des interactions entre un polyélectrolyte négatif et un polyélectrolyte positif. La publication issue du « International Journal of Biological Macromolecules » et intitulée « Recent development of chitosan-based polyelectrolyte complexes with natural polysaccharides for drug delivery » dont les auteurs sont Yangchao Luo et Qin Wang, 64 (2014) 353-367, liste les différents travaux qui ont été effectués avec le chitosan ces dix dernières années pour former de tels complexes ioniques. Les polyanions mélangés avec le chitosan sont l'acide hyaluronique (HA), la pectine, le carrageenan, la gomme de xanthane, la gomme de gellan, la gomme de cashew, la gomme arabique, la carboxymethylcellulose, le glucomannan, et la gomme de kondagogu.

Les hydrogels formés de tels complexes de polyélectrolytes acquièrent également leur forme d'hydrogel par l'intermédiaire d'interactions physiques de plus faible énergie telles que association dipôle-dipôle, liaisons de Van der Waals et hydrogènes. Comparativement aux hydrogels obtenus par réticulation chimique, les hydrogels formés de complexes ioniques ont pour avantage de ne pas être toxiques, bien tolérés par les organismes vivants et d'être biocompatibles. En effet, les réticulants chimiques peuvent induire une certaine toxicité provenant des groupes fonctionnels réactifs qu'ils supportent. Pour éliminer leurs traces dans le gel des étapes de purification des gels implantables ou à usage médical en général, par lavages dans l'eau ou dans des solvants sont donc nécessaires pour les hydrogels réticulés chimiquement. Or ces étapes peuvent dégrader la structure de ces hydrogels. En outre, lorsque ces hydrogels réticulés chimiquement comprennent un principe actif, ces étapes de purification des hydrogels formés peuvent provoquer la perte du principe actif par diffusion dans le milieu qui a servi à extraire les traces d'agent réticulant.

Les gels physiques (réversibles) peuvent être détruits par hausse de la température, par une augmentation de la force ionique, une variation de la polarité du solvant ou du pH, tandis que les gels chimiques sont stabilisés définitivement à travers les liaisons covalentes.

La publication intitulée « Chitosan/cyclodextrin nanoparticles as macromolecular drug delivery system », de A.H.Krauland et M.J.Alonso, International Journal of Pharmaceutics 340 (2007) 134-142 décrit la synthèse de nanoparticules présentées comme des nanogels à base de chitosan et d'un monomère de β-cyclodextrine carboxyméthylée permettant la libération lente de molécules encapsulées. Dans ce cas des nanogels ont été obtenus, et non des gels à l'état macroscopique. Un nano gel peut être défini comme un « objet » de forme globulaire fortement hydraté, de taille souvent comprise entre 20 et plusieurs centaines de nm.

La publication intitulée « Chemically cross-linked and grafted cyclodextrin hydrogels : from nanostructures to drug-eluting medical devices », de A.Concheiro, C.Alvarez-Lorenzo, Advanced Drug Reviews 65 (2013) 1188-1203, décrit la fabrication d'hydrogels à base de cyclodextrines qui sont chimiquement greffées sur des macromolécules préformées (souvent de type poly(acryliques)), obtenus par polymérisation de monomères porteurs de cyclodextrine, ou réticulés par des agents réticulants tels qu'un diépoxyde, un diisocyanate, le dianhydride pyromellitique, le trimetaphosphate de sodium ou l'EDTA.

La publication « Chitosan derivatives bearing cyclodextrin cavities as novel adsorbent matrices », de M.Prabaharan et J.F.Mano, de Carbohydrate Polymers 63 (2006) 153-156 décrit la synthèse de macromolécules de chitosan sur lequel des cyclodextrines sont greffées par l'intermédiaire de liaison amides.

Les agents actifs incorporés dans les hydrogels physiques sont des molécules de haut poids moléculaire (protéines, peptides, ADN...) dont la délivrance contrôlée est rendue possible par leur lente diffusion à travers les réseaux de macromolécules des complexes de polyélectrolytes. La délivrance de ces agents actifs est aussi directement liée à la vitesse d'érosion ou de bio-résorption de la matrice de l'hydrogel.

En revanche, les molécules de faibles poids moléculaires diffusent rapidement à travers le réseau polymère du gel. En effet, si les molécules bioactives ne présentent pas d'interactions particulières avec le ou les polymère(s) formant l'architecture du gel, leur faible encombrement stérique leur permet de diffuser librement à travers les mailles de ces réseaux macromoléculaires très ouverts (un hydrogel étant composé majoritairement d'eau), ainsi la cinétique de libération de ces petites molécules ne sera pas contrôlée par la vitesse de diffusion, ni par la vitesse d'érosion de l'hydrogel et elle sera très rapide.

Les hydrogels physiques connus, ainsi que leurs matériaux alvéolaires correspondants, se désintègrent à court terme (au bout de quelques heures) voire spontanément lors de leur immersion dans l'eau, notamment lorsque celle-ci est chargée en sels ; ce qui est le cas de tous les fluides physiologiques. Cette dernière caractéristique est importante s'agissant d'hydrogels destinés à être implantés dans le corps afin qu'ils conservent leurs structures pendant une durée déterminée afin non seulement d'assurer leur fonction de comblement et/ou de renfort et de permettre une libération contrôlée et ciblée d'un ou plusieurs principe(s) actif(s).

Enfin, il n'existe pas d'hydrogel à ce jour capable de sequestrer ou de contenir une charge minérale sur un site d'implantation (par exemple un défet osseux), ceci est dû à la dégradation trop rapide de la structure de l'hydrogel qui provoque la libération à court terme de la charge minérale de la matrice de l'hydrogel. La charge minérale étant choisie parmi les matériaux utilisés pour le comblement osseux contenant des phosphates de calcium sous formes diverses (poudres, monolytes, granules etc.), en hydroxyapatite ou en phosphate de calcium de constitution similaire au principal constituant minéral de l'os. En effet, l'incorporation d'une quantité de charge minérale suffisante dans un hydrogel et son maintien sur le site d'implantation suffisamment longtemps sont nécessaires dans de nombreuses applications dans le domaine de la chirurgie dentaire, maxillo-faciale ou encore le domaine de la chirurgie orthopédique. Or l'incorporation d'une quantité de charge minérale importante dans un hydrogel réticulé physiquement déstabilise, et facilite la désintégration de dernier.

On connait également US 2010/0221303 A1 et WO 2012/028620 ayant pour objet la formation d'un matériau alvéolaire à partir d'un polymère de polysaccharide.

La présente invention cherche ainsi à proposer un hydrogel, notamment réticulé physiquement, résultant d'un complexe d'au moins un polyélectrolyte négatif et d'au moins un polyélectrolyte positif permettant la complexation de molécules thérapeutiques de faible poids moléculaire (inférieur ou égal à environ 250 g/mol), puis d'assurer leur libération prolongée.

La présente invention cherche également à proposer un hydrogel permettant d'encapsuler, et de contrôler la libération, d'un ou plusieurs composant(s) de poids moléculaires élevés, notamment supérieur à environ 250 g/mol.

La présente invention cherche également à proposer un hydrogel compact, moulable, homogène et thixotrope (afin que ce dernier soit injectable).

La présente invention cherche en outre à proposer un hydrogel et un matériau alvéolaire issu dudit hydrogel qui soient stables et ne se désintègrent pas spontanément lors de leur immersion dans l'eau ou dans un milieu physiologique, et ce quand bien même ils comprennent une quantité importante d'une charge minérale.

La présente invention cherche à proposer un hydrogel ainsi qu'un matériau alvéolaire issu dudit hydrogel qui soient capablent de comprendre une quantité importante de charge minérale, en particulier qui représente jusqu'à 90% de la masse du dispositif, tout en conservant une excellente consistance, une bonne malléabilité et d'excellentes propriétés d'absorption des fluides biologiques (sang) qui permettent d'enclencher le processus biologique de différentiation cellulaire conduisant à la génèse de cellules ostéoblastiques.

### Objet et résumé de l'invention

La présente invention pallie les problèmes précités en ce qu'elle a pour objet, selon un premier aspect, un procédé de fabrication d'un hydrogel comprenant avantageusement les étapes successives suivantes :
- une première étape (i) de fourniture d'au moins une poudre d'un polymère anionique (A) et d'au moins une poudre de chitosan (B) comprenant des fonctions amines (-NH₂) ;
- une seconde étape (ii) consistant à mélanger à sec au moins les poudres (A) et (B) de la première étape pour former un mélange de poudres ;
- une troisième étape (iii) de mise en suspension du mélange de poudres obtenu à l'issue de la seconde étape dans un milieu aqueux, notamment sous agitation, ayant un pH permettant de solubiliser le polymère anionique (A) sans solubiliser le chitosan, de préférence à un pH supérieur ou égal à 4, encore de préférence à un pH supérieur ou égal à 4.5, particulièrement supérieur ou égal à 5 ; de préférence le milieu aqueux est de l'eau (par exemple de l'eau distillée ou de l'eau ultra pure) ou une suspension aqueuse d'au moins une cellule vivante, telles que des cellules humaines ou animales, en particulier des cellules souches, dont le pH est d'environ 7 (+/- 0.5), correspondant au pH d'un milieu de culture ou d'un liquide physiologique ;
- une quatrième étape (iv) d'ajout d'un acide à la suspension obtenue à l'issue de la troisième étape en sorte de former l'hydrogel ;
ou les troisième (iii) et quatrième (iv) étapes sont remplacées par une cinquième étape de mélange (v) comprenant le mélange d'un milieu aqueux acidifié comprenant au moins un composé (C) comprenant au moins une unité d'un hexose ou une unité dérivée d'un hexose, et/ou au moins un phosphate dudit composé (C), avec ledit mélange comprenant au moins les poudres (A) et (B) obtenu à l'issu de la second étape (ii).

Les inventeurs ont découvert que le processus de gélification est strictement lié à la nature chimique des deux polymères (A) et (B), et à la façon particulière de les mettre en oeuvre.

La poudre de chitosan (constituée d'unités monomères glusosamine et N-acétyl glucosamine) est insoluble dans un milieu aqueux ayant un pH supérieur ou égal à 4, de préférence supérieur ou égal à 4.5, particulièrement supérieur ou égal à 5. En revanche, le chitosan se solubilise en milieu aqueux si celui-ci contient un acide dilué tel que l'acide chlorhydrique, l'acide acétique ou l'acide lactique. Dans une solution aqueuse qui est suffisamment acide, les fonctions amines -NH₂ du chitosan sont protonées en fonctions ammonium -NH₃⁺, du fait de cette propriété le chitosan est considéré dans le cadre de la présente invention comme un polycation (polyélectrolyte : PE⁺).

La publication « Chitin and chitosan : Properties and applications » , Mme.Rinaudo, Prog.Polym.Sci.31 (2006) 603-632, décrit la solubilisation du chitosan, laquelle dépend notamment de son degré de désacétylation, de sa masse molaire et du pH du milieu aqueux dans lequel le chitosan est solubilisé.

Il convient de noter qu'à un pH inférieur ou égal à 5 et supérieur ou égal à 4, des fonctions amines du chitosan (B) seront partiellement protonées mais en quantité insuffisante pour obtenir la solubilisation du chitosan (B). Rinaudo rapporte que la solubilisation du chitosan se produit généralement pour un degré d'ionisation des unités glucosamines supérieur à 0.5 (ou 50%).

La proportion d'unités glucosamines ionisées (correspondant au degré d'ionisation du chitosan) peut être évaluée par dosage acido-basique ou par titration colloidale standard bien connus de l'homme du métier.

La mise en suspension du mélange de poudres comprenant les poudres (A) et (B) dans un milieu aqueux engendre une baisse du pH de ce milieu lorsque le polyanion (A) comprend des fonctions acides (par exemples carboxyliques -COOH), puis le pH remonte et se stabilise. Néanmoins, il a été observé que lorsque le pH du milieu aqueux, avant ajout du mélange de poudres, est supérieur ou égal à 4, de préférence supérieur ou égal à 4.5, encore de préférence supérieur ou égal à 5, la baisse éventuelle du pH suite à l'ajout du mélange de poudres n'est pas suffisante pour provoquer la solubilisation du chitosan. Seul l'ajout de l'acide à la quatrième étape entraîne une baisse de pH suffisante permettant de solubiliser le chitosan.

Le polymère anionique selon l'invention, ou polyanion, est soluble à pH supérieur ou égal à 4, en particulier supérieur ou égal à 4.5, notamment supérieur ou égal à 5, et est porteur de fonctions, notamment de fonctions acides sulfates (-O-S(=O)₂-OH ou -SO₄H) et/ou de leurs sels, ou de fonctions acides sulfoniques (-S(=O)₂-OH ou -SO₃H) et/ou de leurs sels, et/ou de fonctions acides phosphoriques (-O-P(=O)₂-OH ou -PO₄H) et/ou de leurs sels, et/ou de fonctions acides carboxyliques (-COOH) et/ou de leurs sels, aptes à former des fonctions anioniques en milieu aqueux, notamment par libération d'un proton H⁺ s'agissant des fonctions acides sulfoniques, sulfates acides, acides phosphoriques ou acides carboxyliques, pour la formation de fonctions carboxylates (-COO⁻) et/ou sulfates -SO₄⁻ et/ou sulfonates -SO₃⁻ et/ou phosphonates -PO₄⁻.

Les macromolécules porteuses des fonctions acides définies ci-dessus, et de fonctions aminées sont ionisables et sont qualifiées de polyélectrolytes. On distingue les polyélectrolytes forts des polyélectrolytes faibles en fonction de la propension qu'ont ces fonctions à s'ioniser en solution aqueuse, laquelle dépend du pKa de chaque polyélectrolyte. La constante d'équilibre Ka est du type (AH) -> <- (A⁻) + (H⁺), (flèches à sens oposés indiquant une réaction équilibrée), tel que Ka = ([A⁻]x[H⁺])/[H⁺] ; ainsi on définit le pKa = - log (Ka) qui permet d'évaluer l'ionisation d'un acide en fonction du pH du milieu. Ainsi le « Polymer Handbook », 2 volumes Set, 4th Edition, classiffie les polyélectrolytes en polyelectrolytes forts et faibles, selon le pKa des fonctions ionisables qu'ils portent. Les groupes sulfates acides, acides sulfoniques et acides phosphoriques sont des polyélectrolytes forts, c'est-à-dire ionisés à bas pH (inférieur à 2), tandis que les polyelectrolytes porteurs de groupes carboxyliques sont des polyanions classés comme polyélectrolytes faibles (car ionisés à pH souvent supérieur à 4). Les polyelectrolytes porteurs d'amines primaires, secondaires et tertiaires sont des polycations classés parmi les polyélectrolytes dit faibles, car ionisés à pH inférieur à 5 (environ).

La formation d'un complexe de polyéléctrolytes se produit par interactions électrostatiques entre les fonctions ioniques acides et basiques. Ainsi, les conditions de pH les plus favorables pour la formation d'un complexe de polyéletrolytes, sont celles lorsque le pH de la solution est supérieur au pKa du polyanion, et inférieur au pKa des fonctions du polycation .

On comprend ainsi au sens de la présente invention par polymère anionique ou polyanion, tout polymère comportant sous forme de poudre des fonctions anioniques ou apte à former des fonctions anioniques une fois solubilisé dans un milieu aqueux, en particulier à un pH supérieur ou égal à 4, plus particulièrement à un pH supérieur ou égal à 4.5, notamment à un pH supérieur ou égal à 5.

Lors de la seconde étape, on mélange intimement les poudres de chitosan (B) (PE⁺) et du polymère anionique (PE⁻) (A). Ainsi les particules de PE⁺ se combinent par interactions électrostatiques aux particules de PE⁻. Ces intéractions électrostatiques s'expliquent par le fait que le chitosan et le polymère anionique présentent des potentiels zétas de signes opposés.

De préférence, le potentiel zeta du chitosan est de l'ordre de +20 mV à +50 mV, et le potentiel zeta du polymère de cyclodextrine (A1) préféré selon l'invention, décrit ci-dessous, est de l'ordre de -30 mV.

Avantageusement, lors de la troisième étape (iii), le chitosan, n'étant pas solubilisé, reste en suspension dans le milieu aqueux grace à la présence du PE⁻ très hydrophile qui reste adsorbé à sa surface par interactions électrostatiques, en formant une couche hydrophile qui permet de maintenir une suspension homogène.

On entend par « suspension homogène » une suspension qui ne sédimente pas rapidement lorsque l'on ralentit ou interrompt l'agitation.

On entend par « poudre », toute substance solide réduite en petits grains à température ambiante.

On entend par « température ambiante », une température comprise entre 10°C et 35°C, en particulier entre 15°C et 25°C.

Les valeurs de pH indiquées dans le présent texte sont mesurées à l'aide d'un pHmètre à température ambiante.

On entend au sens de la présente invention par « polymère », en particulier pour désigner le polymère anionique et le polymère cationique selon l'invention (chitosan (B)), un oligomère ou un polymère pouvant être un homopolymère ou un copolymère (c'est-à-dire comprenant au moins deux unités de répétition différentes).

On entend par « sans solubiliser le chitosan », qu'aucune fonction amine du chitosan ou un nombre insuffisant de fonctions amines du chitosan sont protonnées dans le milieu aqueux pour provoquer une dissolution de la poudre de chitosan. De préférence, la solubilisation du chitosan (B) est obtenu pour un degré d'ionisation alpha au moins de 0.5, c'est-à-dire au moins 50% en nombre des unités glucoses amines du chitosan (B) par rapport au nombre total d'unités glucoses amines du chitosan (B) sont ionisées en unités glucosammonium.

Le degré de protonation à atteindre pour obtenir la solubilisation dépend aussi de la masse molaire et du degré de désacétylation (DDA) du chitosan: si le DDA est faible, il faudra protonner davantage de fonctions amines que s'il est élevé.

De préférence, le degré de désacétylation du chitosan (B) selon l'invention est supérieur ou égal à 60%, encore de préférence supérieur ou égal à 75%, particulièrement inférieur ou égal à 95%, plus particulièrement inférieur ou égal à 90%, notamment inférieur ou égal à 85%.

L'acide ajouté lors de la quatrième étape (iv) ou de l'étape (v) du procédé selon l'invention est différent du polyanion.

Lors de l'ajout de l'acide, le pH de la suspension diminue, particulièrement à un pH inférieur ou égal à 5, plus particulièrement inférieur ou égal à 4.5, notamment inférieur ou égal à 4, ce qui provoque la solubilisation du chitosan par protonation de ses amines (-NH₂) en fonctions ammoniums (-NH₃⁺), avec comme contre-ion le groupe acétate (CH₃COO⁻), ou chlorure (Cl⁻) si l'acide ajouté est respectivement l'acide acétique et l'acide chlorhydrique. Les macromolécules de chitosan se déploient sous l'effet de répulsions électrostatiques intra-moléculaires entre les groupes ammonium formés (les charges électrostatiques de même signe se repoussant), et atteignent une conformation dite « en pelote » qui occupent alors l'intégralité du volume du milieu aqueux. Simultanément se forment des liaisons ioniques entre les groupes ammonium du chitosan (-NH₃⁺) les fonctions anioniques (COO⁻) du polymère anionique.

Puis, une fois les fonctions amines protonées, le pH de l'hydrogel remonte pour se stabiliser, de préférence à un pH supérieur ou égal à 4, encore de préférence à un pH supérieur ou égal à 4.5, en particulier à un pH supérieur ou égal à 5.

Ledit polymère anionique est ainsi dispersé dans le réseau du chitosan. Ces interactions ioniques provoquent la réticulation physique du gel, et la prise en masse de la dispersion aqueuse.

Avantageusement, l'hydrogel selon l'invention est un gel macroscopique qui présente les caractéristiques d'un matériau visco-élastique présentant une forme propre.

De préférence, les caractéristiques relatives au pH du milieu aqueux acidifié suite à la quatrième étape (iv) s'applique également au milieu aqueux de l'étape (v) (comprenant un composé (C) tel que défini dans le présent texte).

De préférence, on entend au sens de la présente invention par « hydrogel », un matériau visco-élastique solide présentant une forme propre, c'est-à-dire qu'il conserve les dimensions et la forme du récipient dans lequel il a été formé lorsqu'on l'extrait de ce récipient (pendant quelques heures ou quelques jours selon les conditions dans lesquelles il est placé).

De préférence, la première étape et/ou la seconde étape et/ou la troisième étape et/ou la quatrième étape et/ou la cinquième étape (v) selon l'invention est/sont effectuées à température ambiante.

Il a été observé que l'ajout de l'acide après la mise en suspension à la troisième étape est très important car cette façon de procéder permet d'obtenir un hydrogel très ferme, stable, et apte à rester sur une zone déterminée sans s'écouler pendant plusieurs heures à température ambiante ou environ à 37°C, notamment pendant au moins 48 heures.

En outre, l'hydrogel selon l'invention conserve sa forme et son intégrité en milieu aqueux, en particulier à température ambiante ou à 37°C environ pendant plusieurs heures, au moins pendant 24 heures, de préférence au moins pendant 48 heures.

Dans l'état de la technique, les hydrogels physiques à base de chitosan sont réalisés en mélangeant la solution aqueuse acide de chitosan avec la solution aqueuse du polyanion ou en saupoudrant le polyanion, par exemple la carboxyméthylcellulose, sur la solution aqueuse acide de chitosan (pH de l'ordre de 2-3). Dans les deux voies, les hydrogels obtenus ne sont pas aussi fermes et stables que l'hydrogel selon l'invention.

Le chitosan dans l'hydrogel selon l'invention a pour rôle d'assurer l'architecture du gel, et de lui conférer ses propriétés rhéologiques requises.

De préférence, le rapport de la masse totale en eau (g) dans l'hydrogel par rapport à la masse totale de l'hydrogel est supérieur ou égal à 50%, encore de préférence supérieur ou égal à 60%, plus préférentiellement supérieur ou égal à 70%, particulièrement supérieur ou égal à 80%, encore plus particulièrement supérieur ou égal à 85%, notamment supérieur ou égal à 90%.

Avantageusement, l'hydrogel selon l'invention est apte à satisfaire à une ou plusieurs propriété(s), dont la ou les suivante(s) : être biocompatible, être injectable au moyen d'une seringue, être capable de conserver une forme déterminée octroyée par moulage pendant au moins quelques heures et/ou ne pas s'écouler afin de rester en place sur la zone sur laquelle il a été appliqué, et être apte à libérer une ou plusieurs substance(s) active(s).

L'hydrogel selon l'invention est avantageusement thixotrope ce qui lui permet d'être injectable. L'hydrogel a ainsi un comportement rhéofluidifiant : le polymère se fluidifie lorsqu'il est exposé à une contrainte mécanique de cisaillement, et récupère son état de gel au repos libre de toute contrainte. Lorsque l'hydrogel est disposé dans le corps de la seringue, il se fluidifie sous l'effet de la contrainte de cisaillement appliquée par l'intermédiaire du piston pour circuler à travers l'orifice de sortie de la seringue, puis dans l'aiguille, et éventuellement dans le cathéter, et enfin récupère sa forme d'hydrogel ferme une fois libre de contrainte sur la zone sur laquelle il a été appliqué.

A titre d'exemple, une aiguille standard en liaison fluidique avec l'orifice de sortie d'une seringue a un diamètre compris entre 0,1 mm et 0,5 mm.

Les hydrogels selon l'invention peuvent être utilisés dans de nombreuses applications médicales, et notamment en tant que matériau de comblement en chirurgies réparatrices, esthétiques, en urologie, en chirurgie dentaire, en ophtalmologie, en orthopédie, en chirurgie maxillo-faciale afin d'empêcher les adhésions de tissus et/ou favoriser la génération d'os ou de tissus mous variés et/ou libérer une ou plusieurs substances active(s) sur la zone sur laquelle ils sont appliqués.

Lorsque les hydrogels selon l'invention comprennent au moins un agent fonctionnel et/ou des cellules vivantes, ils contribuent par exemple à la cicatrisation, au traitement d'une maladie (cancer), à réduire la douleur (effet anesthésiant), à réduire l'inflammation, ou à prévenir ou lutter contre une infection.

Le milieu aqueux lors de la troisième étape (iii) peut également comprendre au moins un composé (C) comprenant au moins une unité d'un hexose ou une unité dérivée d'un hexose et/ou au moins un phosphate dudit composé (C).

On entend, au sens du présent texte, par un composé (C) comprenant au moins une unité d'un hexose ou une unité dérivée d'un hexose, tout composé (C) comprenant au moins une unité de formule brute C₆H₁₂O₆ ou dérivée de ladite formule brute. Il peut s'agir par exemple d'un hexose, ou d'un premier hexose relié par l'intermédiaire d'une liaison glycosidique à un second hexose, identique ou différent au premier hexose (s'agissant d'au moins une unité dérivée d'un hexose).

De préférence, ledit composé comprenant au moins une unité d'un hexose ou une unité dérivée d'un hexose est choisi dans la liste comprenant le glucose (de forme L ou D), le maltose (de forme L ou D), le saccharose, le mannose (de forme L ou D), le fructose (de forme L ou D), le lactose (de forme L ou D), ou leur mélange ; encore de préférence ledit composé est le glucose (de forme L ou D).

On comprend, au sens du présent texte, par un phosphate du composé (C), tout composé comprenant au moins une unité de formule brute C₆H₁₂O₆ ou dérivée de ladite formule brute supportant sur au moins l'un de ses carbones un groupe phosphate (par exemple le groupe phosphoryl (PO₃²⁻)). Il peut s'agir par exemple d'un phosphate d'hexose, ou d'un premier (phosphate d') hexose reliée par l'intermédiaire d'une liaison glycosidique à un second (phosphate d') hexose identique ou différent au premier (phosphate d') hexose (s'agissant d'une unité dérivée d'un (phosphate d') hexose).

En particulier, les phosphates du composé (C) sont des hexose-n-phosphates ou des dérivés d'hexose-n-phosphates (n étant l'un des atomes de carbone numérotés de 1 à 6 de l'unité d'hexose ou du dérivé de l'unité d'hexose supportant un groupe phosphate).

De préférence, un phosphate du composé (C) est choisi parmi le glucose-n-phosphate (de forme L ou D), en particulier, le glucose-6-phosphate ou le glucose-1-phosphate ; le maltose-n-phosphate ; le phosphate de saccharose ; le mannose-n-phosphate ; le fructose-n-phosphate, le lactose-n-phosphate, ou leur mélange ; encore de préférence ledit composé est le glucose-n-phosphate (de forme L ou D).

De préférence, le composé (C) ou le phosphate dudit composé (C) comprend au plus 10 unités d'hexose d'unités dérivées d'hexose, en particulier au plus 5 unités d'hexose d'unités dérivées d'hexose, notamment une ou deux unités d'hexose ou dérivées d'hexose.

Il a été observé de manière surprenante qu'il est possible de mélanger le mélange comprenant au moins les poudres (A) et (B) obtenu à l'issue de l'étape (ii) avec un milieu aqueux acidifié sans qu'il ne soit nécessaire de passer par l'étape de mise en suspension (iii) lorsque ledit milieu aqueux acidifié comprend au moins un composé (C) comprenant au moins une unité d'un hexose ou une unité dérivé d'un hexose et/ou un phosphate dudit composé (C).

Cette disposition permet la formation d'un hydrogel ferme et stable, et ce même une heure après son injection dans un milieu de culture, par exemple le DMEM.

Il a également été observé que la présence du composant (C) à base d'hexose dans le milieu aqueux, et donc au final dans l'hydrogel, permet d'augmenter significativement le pH du milieu aqueux, en particulier à un pH proche de 6, et ainsi d'améliorer très nettement la cytocompatibilité mesurée in vitro.

Les inventeurs ont ainsi découvert de manière surprenante qu'un composé à base d'hexose ou de phosphate d'hexose dans le milieu aqueux acidifié permet d'améliorer la formation d'un gel à base de chitosan et d'un polyanion. L'utilisation du phosphate de glucose est connue en tant qu'agent gélifiant pour améliorer la stabilité des solutions à base de chitosan mais non dans l'amélioration de la formation des hydrogels ioniques à base de chitosan (voir European journal of Pharmaceutics and Biopharmaceutics 88 (2014) 361-373).

Selon un mode de réalisation, la suspension du mélange de poudres obtenue à l'issue de la troisième étape est coulée ou injectée dans un moule puis l'acide est ajouté en sorte de corrélativement mouler et former l'hydrogel.

Selon un mode de réalisation, la seconde étape de mélange des poudres (A) et (B) est effectuée à l'aide d'un dispositif de mélange de poudres, notamment un agitateur planétaire, notamment sans broyage (c'est-à-dire sans réduction significative de la taille des particules solides constituant la poudre).

Dans une variante de réalisation, le milieu aqueux, que ce soit à l'étape (iii) ou à l'étape (v), comprend au moins 0,1% en masse, de préférence au moins 0,5% en masse, encore de préférence au moins 1% en masse, en particulier au moins 2% en masse, plus particulièrement au moins 3% en masse, encore plus particulièrement au moins 4% en masse, notamment au moins 5% en masse, d'au moins un composé (C) comprenant au moins une unité d'un hexose ou une unité dérivé d'un hexose, et/ou d'au moins un phosphate dudit composé (C), par rapport au volume total dudit milieu aqueux.

Dans une variante, la poudre (A) et/ou la poudre (B) à la première étape et/ou le mélange des poudres comprenant au moins les poudres (A) et (B) à la seconde étape est/sont tamisée(s) sur un tamis ayant des mailles dont la taille est inférieure ou égale à 500 µm, de préférence inférieure ou égale à 300 µm, encore de préférence inférieure ou égale à 200 µm, en particulier inférieure ou égale à 150 µm, plus particulièrement inférieure ou égale à 125 µm.

De préférence, la distribution de la taille des particules de la poudre (A) et/ou de la poudre (B) ou du mélange des poudres (A) et (B) a été mesurée avec un Mastersizer S (Malvern Instruments, Orsay, France) en utilisant une lentille de 300 mm. L'échantillon a été dispersé à l'état sec avec de l'air comprimé à 4 bars.

De préférence, la poudre (A) et/ou la poudre (B) et/ou le mélange des poudres (A) et (B), éventuellement en combinaison avec au moins une poudre différente des poudres (A) et (B), telle qu'au moins une charge minérale, est/sont tamisée(s) sur le dispositif de tamisage ayant la référence suivante : un Tamis-Plastique, Nylon DIN 41p5, Nr : 948414 W : 0.125µm, commercialisé par Bioblock Scientific.

Le respect d'une taille déterminée pour les particules de polycation et de polyanion est important car elle permet la formation d'un gel homogène, c'est-à-dire sans présence de grumeaux, et présentant les propriétés visco-élastiques attendues.

La poudre (A) et la poudre (B) peut/peuvent être tamisée(s) également à l'aide d'une tamiseuse à vibration, par exemples celles commercialisées par la société Fristch.

De préférence, lors de la première étape, la poudre (A) et/ou la poudre (B) est/sont broyée(s) séparément préalablement à leur passage sur un tamis, de préférence à l'aide d'un dispositif Pulverisette 14® commercialisé par la société Fritsch et équipé d'une grille de 200 µm.

Dans une variante, le mélange de la seconde étape est effectué par co-broyage à sec au moins des poudres (A) et (B).

De préférence, les poudres (A) et (B) sont co-broyées à sec dans un mortier manuellement.

Les poudres (A) et (B) peuvent être également co-broyées sur un broyeur à billes, notamment un micro-broyeur planétaire pulvérisette tels que ceux commercialisés par la société Fritsch.

Le broyage permet la réduction de la taille des particules solides en fragments plus petits. Le terme de co-broyage fait référence au broyage simultané d'au moins deux poudres (A) et (B) différentes. Dans les variantes ci-dessus, il a été observé que le co-broyage et/ou le broyage suivie d'un mélange permettent avantageusement de réduire la taille des particules solides des poudres mais également d'assurer un mélange intime entre les poudres (A) et (B) et ainsi une meilleure surface de contact entre le polymère anionique et le chitosan. Cette disposition favorise l'homogénéité de la dispersion aqueuse comprenant ces poudres à la troisième étape. La reproductibilité dans la formation de l'hydrogel est ainsi également accrue, tout comme la réactivité entre le polymère anionique et le chitosan, en particulier pour la prise en masse de l'hydrogel.

Dans une variante, le polymère anionique comprend des fonctions sulfates acides (-O-S(=O)₂-OH ou -SO₄H) et/ou des sels dites fonctions sulfates acides, et/ou de fonctions acides sulfoniques (-S(=O)₂-OH ou -SO₃H) et/ou des sels dites fonctions acides sulfoniques, et/ou des fonctions acides phosphoriques (-O-P(=O)₂-OH ou -PO₄H) et/ou des sels desdites fonctions acides phosphoriques, et/ou de fonctions acides carboxyliques (-COOH) et/ou des sels dites fonctions acides carboxyliques, de préférence des fonctions acides phosphoriques et/ou des sels desdites fonctions acides phosphoriques; et/ou des fonctions acides carboxyliques (-COOH) et/ou des sels desdites fonctions acides carboxyliques, encore de préférence des fonctions acides carboxyliques (-COOH) et/ou des sels desdites fonctions acides carboxyliques, particulièrement des fonctions acides carboxyliques (-COOH).

Les sels des acides sulfoniques (sels sulfonates -SO₃X), des sulfates acides (sels de sulfates - SO₄X), des acides phosphoriques (sels phosphonates -PO₄X) et des acides carboxyliques (sels carboxylates -COOX) sont monovalents, lesdits sels comprennent ainsi au moins un groupe -OX (en remplacement du groupe OH présent dans les acides) dans lequel X est de préférence choisi parmi le sodium, le potassium, le lithium, l'argent, encore de préférence il s'agit du potassium ou du sodium.

Dans une variante, le polymère anionique est choisi dans la liste comprenant : un polymère de cyclodextrine, l'acide hyaluronique, la carboxyméthylcellulose, l'héparine, un sel d'héparinate, notamment l'héparinate de sodium, l'acide polyacrylique, le polyacrylate de sodium, un sel d'acide polyacrylique, la pectine, l'acide alginique, l'alginate de sodium, le carrageenan, la gomme de xanthane, la gomme de gellan (sel d'acide carboxylique), le glucomannan, la gomme de kondagogu, la gomme arabique, la gomme de cash, encore de préférence dans la liste constituée par un polymère de cyclodextrine, l'acide hyaluronique, la carboxyméthylcellulose, l'héparine, la pectine, l'acide alginique, l'alginate de sodium, l'acide acrylique et un sel d'acide acrylique, plus préférentiellement un polymère de cyclodextrine, l'acide acrylique, la pectine et l'alginate de sodium.

L'héparine, un sel d'héparinate, le carrageenan comprennent des fonctions sulfates acides ou des sels des fonctions sulfates basiques.

L'acide hyaluronique, la pectine, le polymère d'acide acrylique ou son sel, la carboxymethylcellulose, l'acide alginique, la gomme de xanthane, la gomme de gellan, le glucomannan, la gomme de kondagogu, la gomme arabique, la gomme de cash et le polymère de cyclodextrine (selon sa voie de synthèse) comprennent des fonctions acides carboxyliques (-COOH) ou des sels desdites fonctions acides carboxyliques (lesquelles sels ont déjà été définis dans le présent texte au paragraphe ci-dessus en référence au polymère anionique).

Dans une variante, le polymère anionique (A) est un polymère de cyclodextrine obtenu par la réaction de polymérisation d'un mélange comprenant au moins une cyclodextrine et/ou au moins un dérivé de cyclodextrine et/ou au moins un complexe d'inclusion de cyclodextrine et/ou au moins un complexe d'inclusion dérivé de cyclodextrine, avec
- au moins un acide (poly)carboxylique et/ou son anhydride d'acide (poly)carboxylique correspondant (liste A1) ; ou
- au moins un pyrophosphate (liste A2); ou
- au moins l'épichlorhydrine, le polymère obtenu subissant une étape de carboxyalkylation, en particulier carboxyméthylation, en sorte de greffer des fonctions acides carboxyliques (-COOH) (liste A3).

Avantageusement, le réseau hydrophile du polymère de cyclodextrine augmente la biocompatibilité et assure la cohésion de l'hydrogel qui ne peut se dissoudre spontanément dans le milieu physiologique et migrer. Le réseau hydrophile apporte aussi une stabilisation du complexe d'inclusion entre l'invitée et la cavité de la cyclodextrine. La cyclodextrine contribue aussi à la stabilité du gel en améliorant ses propriétés d'adsorption (dites propriétés réservoir) régulées par l'affinité hote invitée, et provoquant la libération plus ou moins ralentie de cette dernière.

L'hydrogel peut comprendre un polymère de cyclodextrine soluble ou un polymère de cyclodextrine insoluble ou un mélange de ces derniers, de préférence un polymère de cyclodextrine soluble, encore plus particulièrement appartenant à la famille (A1).

Le polymère de cyclodextrine anionique, assure à la fois la réticulation du chitosan par intéractions ioniques, et permet en même temps la complexation de molécules thérapeutiques de faible poids moléculaire (inférieur à environ 250 g/mol), par formation de complexes d'inclusions, puis d'assurer leur libération prolongée.

Le polymère de cyclodextrine selon l'invention, en particulier réticulé ou hyperbranché, notamment de la liste (A1), peut exister sous forme soluble ou insoluble. Le polymère soluble est constitué de gels de dimensions nanométriques (c'est-à-dire ayant des dimensions inférieures ou égales à environ 100nm) (voir publication Green Chemistry, 2015, 17, 2444-2454 représentant des photos de nanogels globulaires à base d'un polymère de cyclodextrine), qui forment des solutions limpides dans l'eau. Le polymère insoluble correspond à des particules micrométriques (c'est-à-dire dont les dimensions sont supérieures à 1 µm), millimétriques et au-delà, qui forment des systèmes biphasiques dans l'eau, les solutions sont donc troubles et non limpides.

De préférence, le polymère de cyclodextrine est celui appartenant à la liste (A1). Cette famille de polymères (A1) a pour avantage d'être plus facile à mettre en suspension dans un milieu aqueux, car ces polymères sont très hydrophiles. Cette disposition facilite ainsi la formation d'un hydrogel homogène et ferme.

Avantageusement, le polymère de cyclodextrine, en particulier ceux appartenant à la liste (A1), conserve sa fonction de rétention et de libération contrôlée de molécules, notamment de petites tailles, ce qui n'est pas possible avec le chitosan. Le chitosan est complémentaire puisque des molécules de grande taille peuvent être retenues à l'intérieur de son réseau macromoléculaire tridimensionnel, puis libérées de façon plus ou moins prolongée.

D'autres polymères de cyclodextrines existent que ceux de la liste (A1) définis ci-dessus, toutefois ces derniers sont très spécifiques car leurs très hauts taux de fonctions carboxylates (au moins 3mmol de groupes COOH par gramme, notamment environ au moins 3-5 mmoles de groupes COOH par gramme) ne sont pas atteignables par les autres voies de polymérisation ou de modification chimique de polymères de cyclodextrines. La famille de polymères de la liste (A1) définie ci-dessus permet ainsi d'obtenir des hydrogels ayant une forme propre, c'est à dire aptes à être moulés, taillés, ayant une texture ferme, homogène et durable.

De préférence, le polymère de cyclodextrine, notamment appartenant à la liste (A1), est obtenu à partir d'un mélange de base dans lequel le rapport du poids (g) en cyclodextrine(s) et/ou dérivé(s) de cyclodextrine et/ou complexe(s) d'inclusion (dérivé(s)) de cyclodextrine par rapport au poids total (g) dudit mélange de base est supérieur ou égal à 40%, de préférence inférieur ou égal à 65%.

On entend par « cyclodextrine », toute cyclodextrine native, en particulier l'a-cyclodextrine ou la β-cyclodextrine ou la gamma-cyclodextrine.

On entend par « dérivé de cyclodextrine », toute cyclodextrine native, en particulier l'a-cyclodextrine ou la β-cyclodextrine ou la gamma-cyclodextrine, dont au moins un groupe hydroxyle, de préférence au moins la moitié des groupes hydroxyles, est/sont substitué(s), en particulier aminé(s), notamment par une fonction amine (-NH₂), estérifié(s), éthérifié(s), alkylé(s), hydroxyalkylé(s), carboxyalkylé(s), en particulier carboxyméthylé(s), sulfoalkylé(s), en particulier sulfobutylé(s), et leurs mélanges.

Ledit au moins un groupe hydroxyle de la cyclodextrine peut être substitué par un chaine alkyle, linéaire et/ou ramifiée, insaturée ou non, substituée ou non, comprenant de 1 à 10 atomes de carbone, de préférence de 1 à 6 atomes de carbones, encore de préférence de 1 à 4 atomes de carbone, notamment un groupe méthyle, un groupe éthyle, un groupe n-propyl, un groupe isopropryl, un groupe n-butyl, un groupe iso-butyl, un groupe sec-butyl, un groupe ter-butyle, chacun desdits groupes étant éventuellement, et ce indépendamment les uns des autres, substitué par un ou plusieurs groupes hydroxyles (-OH) ; et/ou substitué par un ou plusieurs groupes acides carboxyliques (-COOH) ; et/ou substitué par un ou plusieurs groupes acides sulfoniques et/ou un ou des sels desdites fonctions acides sulfoniques. Ledit au moins un groupe hydroxyle de la cyclodextrine peut ainsi être substitué par un groupe hydroxypropyl ou par un groupe carboxyméthyl.

On entend par « complexe d'inclusion de cyclodextrine » ou « complexe d'inclusion dérivé de cyclodextrine », toute cyclodextrine ou dérivé de cyclodextrine au sens de la présente invention complexant un agent fonctionnel tel que défini dans le présent texte, ou complexant une cellule vivante telle que définie dans le présent texte.

L'homme du métier sélectionne une ou plusieurs cyclodextrine(s) et/ou un ou plusieurs dérivé(s) de cyclodextrine et/ou un ou plusieurs complexe(s) d'inclusion (dérivés) de cyclodextrine apte(s) à réagir avec l'agent réticulant. En particulier, s'agissant de la liste A1 des polymères de cyclodextrine, si un ou des dérivés de cyclodextrine est/sont utilisé(s), il(s) doi(ven)t présenter suffisamment de groupes hydroxyles (-OH) pour être aptes à effectuer une réaction de polycondensation avec les acides (poly)carboxyliques et/ou leurs anhydrides correspondants.

EP 1 165 621 B1 décrit la synthèse de polymères de cyclodextrine obtenus par réactions de polycondensation entre des acides (poly)carboxyliques et les cyclodextrines natives (alpha, béta, gamma) ou dérivées (méthyl, hydroxypropyl cyclodextrines) convenant parfaitement à la mise en œ uvre de l'invention. Le polymère obtenu est soit soluble dans l'eau, soit insoluble selon l'avancement de la réaction de polycondensation. Il se caractérise par une forte teneur en cyclodextrine, comprise entre 40% et 65 % en masse et par sa richesse en fonctions acides carboxyliques, de l'ordre de 3-5 mmol par gramme de polymère de cyclodextrine.

On comprend au sens de la présente invention par « acide carboxylique » un acide comprenant une fonction -COOH.On comprend au sens de la présente invention par « acide polycarboxylique », un acide comprenant au moins deux fonctions -COOH, et par « son anhydride correspondant », un acide comprenant au moins une fonction -CO-O-CO-

On entend par « pyrophosphate », tout composé comprenant une liaison P-O-P, en particulier comprenant la fonction suivante -O-PO(OH)-O-PO(OH)-O-.

Selon un mode de réalisation, le polymère de cyclodextrine obtenu par réaction avec l'épichlorhydrine ne comprend pas de fonctions acides carboxyliques de sorte qu'il subit une réaction de carboxyalkylation, notamment de carboxyméthylation à l'aide de l'acide chloroacétique en sorte de greffer des groupes -CH₂COOH sur ledit polymère.

La carboxyalkylation est de préférence effectuée avec un acide (poly)carboxylique substitué par au moins un atome de chlore, de brome ou d'iode sur sa chaîne alkyle. De préférence, la chaîne alkyle est linéaire et/ou ramifiée, insaturée ou non, substituée ou non, comprenant de 1 à 10 atomes de carbone, de préférence de 1 à 6 atomes de carbones, encore de préférence de 1 à 4 atomes de carbone, notamment un groupe méthyle, un groupe éthyle, un groupe n-propyl, un groupe isopropryl, un groupe n-butyl, un groupe iso-butyl, un groupe sec-butyl, un groupe ter-butyle.

Dans une variante, le pyrophosphate (A2) est le métatriphosphate de sodium.

Dans une variante, l'acide (poly)carboxylique ou son anhydride d'acide correspondant est choisi dans la liste comprenant : les acides (poly)carboxyliques saturés ou insaturés ou aromatiques, linéaires ou branchés ou cycliques et les acides hydroxypoly(carboxyliques), de préférence dans la liste comprenant : l'acide citrique, l'acide polyacrylique, l'acide poly(méthacrylique), l'acide 1,2,3,4-butanetétracarboxylique (BTCA), l'acide maléique, l'acide citraconique, l'acide itaconique, l'acide 1,2,3-propanetricaboxylique, l'acide trans-aconitique, l'acide all-cis-1,2,3,4-cydopentanetétracarboxylique, l'acide méllitique, l'acide pyroméllitique, l'acide éditique (acide éthylene diamine tétraacétique ou EDTA), l'acide oxydisuccinique, l'acide thiodisuccinique ou parmi les anhydrides acides desdites acides (poly)carboxyliques précités, tel que le dianhydride pyroméllitique, et leurs mélanges, de préférence l'acide citrique et le BTCA.

De préférence, l'acide (poly)carboxylique est choisi parmi les acides acycliques, encore de préférence ne comprenant pas de fonctions amines (-NH₂), plus particulièrement parmi les acides exemptes d'azote. Les polymères de cyclodextrine obtenus avec ces acides sont plus hydrophiles que ceux obtenus avec des acides cycliques et sont synthétisables par voie aqueuse contrairement par exemple aux polymères obtenus avec le dianhydride pyromméllitique dont la synthèse doit être effectuée dans le DMF (diméthylformamide).

Dans une variante, l'acide ajouté à la suspension lors de la quatrième étape ou utilisé pour acidifier le milieu aqueux lors de l'étape (v) du procédé selon l'invention est choisi dans la liste comprenant : l'acide acétique, en particulier l'acide acétique glacial, l'acide formique, l'acide tartrique, l'acide salicylique, l'acide glutamique, l'acide propanoïque, l'acide chlorhydrique, l'acide citrique, l'acide lactique, et leur mélange ; de préférence l'acide acétique, l'acide chlorhydrique, l'acide lactique et leur mélange, encore de préférence l'acide acétique et l'acide chlorhydrique.

De préférence, ledit acide acétique est concentré à au moins 50%, encore de préférence à au moins 80%, plus préférentiellement à au moins 95%, notamment à au moins 99% (acide acétique « pur » dit « glacial »)

De préférence, de manière général, au moins 0,5%, encore de préférence au moins 1% en volume d'un acide est ajouté au milieu aqueux à l'étape (iii) ou à l'étape (v) par rapport au volume total dudit milieu aqueux.

Dans une sous-variante, l'acide ajouté à la suspension lors de la quatrième étape (iv) ou de l'étape (v) du procédé selon l'invention est choisi parmi l'acide chlorhydrique, l'acide acétique et l'acide lactique, encore de préférence parmi l'acide lactique et l'acide chlorhydrique, en particulier l'acide lactique.

Il a été observé que la fermeté et la stabilité du cordon d'hydrogel injecté sont meilleures avec ces acides, en particulier avec l'acide lactique.

De plus, les acides chlorhydrique et lactique ont l'avantage d'être inodores comparativement à l'acide acétique.

Avantageusement, il a également été observé que l'acide chlorhydrique, l'acide acétique et l'acide lactique, en particulier l'acide lactique, notamment lorsqu'il est combiné avec au moins un composé comprenant au moins une unité d'un hexose ou une unité dérivée d'un hexose dans le milieu aqueux, permettent d'obtenir des bons résultats en terme de cytocompatibilité in vitro (test cellules MC3T3).

De préférence, l'acide lactique ajouté est une solution aqueuse comprenant au moins 85% en masse d'acide lactique pur par rapport au volume de ladite solution aqueuse.

Dans une variante, le polymère de cyclodextrine anionique (A) comprend au moins 2 mmoles/g de fonctions acides carboxyliques (-COOH), de préférence au moins 3 mmoles/g de fonctions acides carboxyliques (-COOH), encore de préférence au moins 3,5 mmoles/g de fonctions acides carboxyliques (-COOH).

La quantité de fonctions carboxyliques par g de polymère de cyclodextrine peut être mesurée par titrage acido-basique en présence d'indicateurs colorés.

La méthode de mesure consiste à mettre en solution une quantité déterminée d'un polymère de cyclodextrine dans un volume donné d'eau en présence d'un indicateur coloré, par exemple la phénolphtaléine (quelques gouttes), puis d'ajouter progressivement (au goutte à goutte), notamment à l'aide d'une burette graduée, une solution de soude à 0,1 mol/litre à ladite solution sous agitation (à l'aide d'un barreau aimanté par exemple) jusqu'à obtenir un changement de couleur de la solution contenant l'indicateur coloré. Le nombre de moles de soude ajouté correspondant ainsi au nombre de moles de fonctions acides carboxyliques (-COOH) neutralisées.

Dans une variante, le rapport de la masse (g) de ladite au moins une poudre de chitosan (B), par rapport à la masse totale (g) de l'hydrogel à l'issue de la quatrième étape (iv) ou l'issue de la cinquième étape (v) est supérieur ou égal à 1%, de préférence inférieur ou égal à 8%, encore de préférence inférieur ou égal à 5%, plus préférentiellement inférieur ou égal à 4%.

Dans une variante, le rapport de la masse (g) de ladite au moins une poudre du polymère anionique (A) par rapport à la masse totale (g) de l'hydrogel à l'issue de la quatrième étape (iv) ou l'issue de la cinquième étape (v) est supérieur ou égal à 1%, de préférence inférieur ou égal à 20%, encore de préférence inférieur ou égal à 15%, plus préférentiellement inférieur ou égal à 10%, particulièrement inférieur ou égal à 6%.

Dans une variante, le ratio de la masse (g) de la poudre de chitosan (B) sur la masse (g) de la poudre de polyanion (A) est compris entre 0,6 et 1,4, encore de préférence compris entre 0,8 et 1,2, plus préférentiellement compris entre 0,9 et 1,1.

Dans une variante, le procédé de fabrication d'un hydrogel selon l'invention comprend l'ajout d'au moins une charge minérale sous forme de poudre au mélange de poudres réalisé lors de la seconde étape, de préférence le rapport de la masse (g) de charge minérale par rapport à la masse totale (g) du mélange de poudres est supérieure ou égale à 30%.Ladite au moins une charge minérale est donc mélangée intimement aux poudres (A) et (B).

De préférence, ladite charge minérale est tamisée et/ou (co)broyée selon les variantes de réalisation définies ci-dessus s'agissant des poudres (A) et (B), en particulier s'agissant de la taille des particules tamisée.

Dans une variante, ladite au moins une charge minérale est choisie dans la liste comprenant : les biocéramiques, en particulier les phosphates de calcium, tels que l'hydroxyapatite et le bétatriphosphate de calcium, le carbonate de calcium, l'alumine (Al₂O₃), la zircone (ZrO₂), les verres, les verres ionomères, le dioxyde de titane et un mélange de ces derniers (pour la reconstruction osseuse).

Dans une variante, le milieu aqueux à l'étape (iii) ou à l'étape (v) comprend au moins un agent fonctionnel et/ou au moins une cellule vivante.

Ledit agent fonctionnel est en particulier un agent bioactif, plus particulièrement une molécule, une macromolécule ou un micro-organisme qui présente une activité thérapeutique. Cette définition s'applique également dans la suite du présent texte.

Avantageusement, ledit agent fonctionnel est choisi dans une première liste (I) comprenant les anticoagulants, les anti-thrombogéniques, les agents antimitotiques, les agents anti-prolifération, les agents anti-adhésion, les agents anti-migration, les promoteurs d'adhésion cellulaire, les facteurs de croissance, les molécules antiparasitaires, les agents anti-inflammatoires, les vitamines, les hormones, les protéines, les antifongiques, les molécules antimicrobiennes, les antiseptiques, les antibiotiques (notamment ciprofloxacine et la chlorhexidine), la nicotine, une ou plusieurs huiles essentielles, et le mélange de ces derniers ; et ladite au moins une cellule vivante est choisie dans une seconde liste (II) comprenant les cellules animales et/ou humaines et/ou végétales.

De préférence, les cellules vivantes comprennent les cellules prokaryotiques, les cellules eukaryotiques, des bactéries, des levures, des cellules végétales, des cellules animales, telles que des cellules endothéliales, des cellules nerveuses, des lymphoblastes, des fibroblastes, des ostéoblastes, des hépatocytes, des cellules souches, des cellules embryonnaires, des adipocytes,... ; et des cellules conçues pour exprimer une molécule particulière.

La présente invention a également pour objet, selon un second aspect, un procédé de fabrication d'un matériau alvéolaire, en particulier un matériau spongieux, telle une éponge, comprenant un procédé de fabrication d'un hydrogel décrit selon l'une quelconque des variantes de réalisation précédentes en référence à un premier aspect de l'invention, pour l'obtention de l'hydrogel, et comprenant en outre une étape de lyophilisation effectuée sur l'hydrogel obtenu à l'issue de la quatrième étape (iv) ou de l'étape (v) pour la formation d'un matériau alvéolaire.

De préférence, l'étape de lyophilisation est une étape qui permet la dessiccation d'un composé hydraté par congélation puis par sublimation de la glace sous atmosphère réduite.

Dans une variante, un agent porogène est ajouté sous forme de poudre au mélange de poudres lors de la seconde étape (ii) du procédé de fabrication de l'hydrogel.

Un tel agent porogène peut être tout agent apte à être transformé en gaz dans des conditions acides, pH inférieur ou égal à 5, dont les pores sont formés par les molécules de dioxydes de carbone qui s'échappe de l'hydrogel. De tels agents porogènes peuvent être du carbonate d'ammonium, du bicarbonate d'ammonium, du carbonate de sodium, du bicarbonate de sodium, du carbonate de calcium, et leur mélange. La proportion en poids d'agent porogène est comprise entre 1% et 15% par rapport au poids total du mélange de poudres à la seconde étape. Ces composés sont disponibles chez Sigma-Aldrich.

Dans l'état de la technique, le matériau alvéolaire est désigné sous le terme de « porous scaffold ».

La concentration en agent porogène affecte la taille des pores dans le matériau alvéolaire. La taille moyenne des pores dans le matériau alvéolaire est de préférence supérieure ou égale à 1 µm et inférieure ou égale à 1000 µm, encore de préférence entre 100-500 µm. La densité des pores est comprise entre 4% et 75%.

De préférence, le matériau alvéolaire poreux est souple et élastique, c'est-à-dire qu'il peut être comprimé ou étiré manuellement dans toutes directions sans être endommagé (en particulier sans se déchirer), puis tend à reprendre ses dimensions initiales (ou proches de ses dimensions initiales) après relâchement de la compression ou de l'étirement.

De préférence, le matériau alvéolaire comprend des pores fermés.

De préférence le matériau alvéolaire a une structure spongieuse, telle qu'une mousse.

Cette étape de lyophilisation peut être effectuée avec n'importe quel appareil de l'état de la technique. La lyophilisation est effectuée pendant suffisamment de temps pour ôter au moins 98% d'eau, au moins 99% d'eau, encore de préférence au moins 99,5% d'eau.

Avantageusement, cette étape de lyophilisation est effectuée après avoir donné à l'hydrogel une forme déterminée, par exemple en disposant ledit hydrogel dans un moule, puis en lui faisant subir une étape préalable de congélation.

Dans une variante, le procédé de fabrication d'un matériau alvéolaire comprend une étape de traitement thermique dudit matériau alvéolaire à une température supérieure ou égale à 100°C, pendant au moins 5 minutes, notamment pendant au moins 60 minutes, de préférence à une température supérieure ou égale à 120°C pendant au moins 5 minutes, notamment pendant au moins 30 minutes, encore de préférence à une température supérieure ou égale à 140°C pendant au moins 5 minutes.

Cette étape de traitement thermique est effectuée sur le matériau alvéolaire obtenu, en particulier après l'étape de lyophilisation.

Cette disposition améliore très significativement les propriétés mécaniques du matériau alvéolaire ainsi que son élasticité et sa tenue en milieu aqueux, en particulier en réduisant sa vitesse de solubilisation.

Avantageusement, il a été observé que la résistance en compression est également améliorée. Le matériau alvéolaire se comprime sous l'effet d'une contrainte en compression exercée manuellement mais tend à retrouver ses dimensions et sa forme initiales rapidement une fois libéré de cette contrainte. Une telle qualité a été observée sur le matériau alvéolaire sec, mais aussi après imprégnation dudit matériau alvéolaire dans l'eau.

Dans une variante, ledit procédé comprend une étape d'imprégnation du matériau alvéolaire dans une solution comprenant au moins un agent fonctionnel et/ou au moins une cellule vivante.

Ledit agent fonctionnel est en particulier un agent bioactif.

Avantageusement, ledit agent fonctionnel est choisi dans une première liste (I) comprenant les anticoagulants, les anti-thrombogéniques, les agents antimitotiques, les agents anti-prolifération, les agents anti-adhésion, les agents anti-migration, les promoteurs d'adhésion cellulaire, les facteurs de croissance, les molécules antiparasitaires, les agents anti-inflammatoires, les vitamines, les hormones, les protéines, les antifongiques, les molécules antimicrobiennes, les antiseptiques, les antibiotiques (notamment ciprofloxacine et la chlorhexidine), la nicotine, une ou plusieurs huiles essentielles, et le mélange de ces derniers ; et ladite au moins une cellule vivante est choisie dans une seconde liste (II) comprenant les cellules animales et/ou humaines et/ou végétales.

De préférence, les cellules vivantes comprennent les cellules prokaryotiques, les cellules eukaryotiques, des bactéries, des levures, des cellules végétales, des cellules animales, telles que des cellules endothéliales, des cellules nerveuses, des lymphoblastes, des fibroblastes, des ostéoblastes, des hépatocytes, des cellules souches, des cellules embryonnaires, des adipocytes,... ; et des cellules conçues pour exprimer une molécule particulière.

La présente invention a pour objet, selon un troisième aspect, un dispositif d'absorption et de drainage et/ou de libération d'un agent fonctionnel et/ou de support d'au moins une cellule vivante comprenant :
- l'hydrogel obtenu par la mise en œuvre du procédé décrit selon l'une quelconque des variantes de réalisation en référence au premier aspect selon l'invention ; ou
- comprenant le matériau alvéolaire obtenu par la mise en œuvre du procédé décrit selon l'une quelconque des variantes de réalisation en référence au second aspect selon l'invention.

Avantageusement, ledit dispositif est choisi parmi : une seringue comprenant au moins un réservoir recevant un volume déterminé d'hydrogel à injecter ; un pansement apte à délivrer au moins un agent fonctionnel et/ou apte à drainer une plaie, et un patch apte à délivrer au moins un agent fonctionnel, tel que la nicotine.

Ledit agent fonctionnel est en particulier un agent bioactif.

La présente invention a pour objet, selon un quatrième aspect, un dispositif permettant la mise en œuvre du procédé selon l'une quelconque des variantes selon un premier aspect de l'invention pour la fabrication d'un hydrogel, comprenant une première seringue recevant le mélange comprenant au moins les poudres (A) et (B) obtenu à l'issue de l'étape (ii) et une seconde seringue recevant un volume déterminé d'un milieu aqueux acidifié comprenant au moins un composé (C) comprenant au moins une unité d'un hexose ou une unité dérivée d'un hexose, et/ou au moins un phosphate dudit composé (C), ledit dispositif comprenant un organe de mise en liaison fluidique des première et seconde seringue agencé en sorte de permettre le mélange du milieu aqueux acidifié comprenant au moins un composé (C) comprenant au moins une unité d'un hexose ou une unité dérivée d'un hexose, et/ou au moins un phosphate dudit composé (C) avec ledit mélange comprenant au moins les poudres (A) et (B).

Ledit organe de mise en liaison fluidique est solidarisé de manière amovible ou non à la première seringue et à la seconde seringue, de préférence il s'agit d'un d'organe amovible.

Avantageusement, les première et seconde seringues sont en liaison fluidique au niveau de leurs embouts d'injection par l'intermédiaire d'un organe de mise en liaison fluidique (par exemple des connectiques type Lueur) ce qui permet d'introduire à l'aide du piston de la seconde seringue, le milieu aqueux acidifié comprenant au moins le composé (C) dans la première seringue, et inversement via le piston de la première seringue.

Les pistons sont actionnés de multiples fois jusqu'à la formation d'un hydrogel, le cisaillement généré favorisant la dissolution de la poudre de chitosan et les interactions ioniques avec le polymère anionique pour provoquer la formation d'un hydrogel stable et ferme.

La présente invention a pour objet, selon un cinquième aspect, un hydrogel susceptible d'être obtenu par la mise en œuvre du procédé de fabrication selon l'une quelconque des variantes de réalisation en référence au premier aspect selon l'invention, comprenant avantageusement :
- un polymère de cyclodextrine (A) obtenu par la réaction de polymérisation d'un mélange comprenant au moins une cyclodextrine et/ou au moins un dérivé de cyclodextrine et/ou au moins un complexe d'inclusion de cyclodextrine et/ou au moins un complexe d'inclusion dérivé de cyclodextrine, avec
- -au moins un acide (poly)carboxylique et/ou son anhydride d'acide (poly)carboxylique correspondant (A1) ; ou
- au moins un pyrophosphate (A2) ;ou
- au moins l'épichlorhydrine, le polymère obtenu subissant ensuite une étape de carboxyméthylation en sorte de greffer des fonctions acides carboxyliques (-COOH) (A3), et
- le chitosan (B) comprenant des fonctions amines (NH₂).

De préférence, le polymère de cyclodextrine appartient à la famille (A1).

Dans une variante, le polymère anionique (A) comprend des fonctions acides carboxyliques (-COOH).

Dans une variante, le polymère anionique (A) comprend au moins 3 mmoles/g de fonctions acides carboxyliques (COOH).

De préférence, les variantes de réalisation et définitions décrites ci-dessus en référence à l'un quelconque des aspects un à quatre caractérisant l'hydrogel s'applique indépendamment à l'hydrogel selon un cinquième aspect de l'invention.

### Description détaillée des dessins :

- les figures 1A et 1B représentent un hydrogel qui a été obtenu par un procédé différent du procédé de fabrication d'un hydrogel selon l'invention ;
- les figures 2A et 2B représentent un hydrogel obtenu par la mise en œuvre du procédé de fabrication ;
- la figure 3 représente les courbes des modules élastiques G' de matériaux alvéolaires selon l'invention ;
- la figure 4 est un histogramme représentant en ordonnée la quantité de ciprofloxacine (CFX) absorbée en mg par g de différents matériaux alvéolaires ;
- la figure 5 est un graphique représentant les courbes de libération de ciprofloxacine en fonction du temps de différents matériaux alvéolaires correspondant à ceux référencés à la figure 4 ;
- la figure 6 représente les graphiques des évolutions des modules élastique et visqueux G' et G" d'un exemple d'hydrogel selon l'invention en fonction du temps (minutes) et de deux contraintes A et B appliquées de façon alternée ;
- les figures 7 et 8 sont des photographies représentant des hydrogels selon l'invention respectivement 1 heure et 24 heures après leur formation.

### Description détaillée de l'invention

La présente invention sera mieux comprise à la lecture des exemples de réalisation décrits ci-après et cités à titre non limitatif.

Les composés utilisés les suivants :
- poudre de chitosan (CHT1): « Chitosan - Medium molecular weight » (Médium Mw : 431 000 g/mol) vendu par Sigma Aldrich, n° de cas : 9012-76-4, sous forme de poudre, la viscosité est de 563.00 cps à une concentration de 1% dans de l'acide acétique à 1%, le degré de désacétylation est supérieur ou égal à 75%, notamment d'environ 75%.
- poudre de chitosan (CHT2) : « Chitosan - Low molecular weight » (LowMw : 39 000 g/mol) vendu par Sigma Aldrich, n° de cas : 9012-76-4, sous forme de poudre et/ou granules, la viscosité est de 96 cps à une concentration de 1% dans de l'acide acétique à 1%, le degré de désacétylation est supérieur ou égal à 75%.
- poudre de chitosan (CHT3) : « Chitosan - High molecular weight » (HighMw : 1 250 000 g/mol) vendu par BioResources, n° de cas : 9012-76-4, sous forme de poudre et/ou granules, le degré de désacétylation est supérieur ou égal à 75%.
- polymère de cyclodextrine : le polymère est synthétisé de la façon suivante : 400 g de β-cyclodextrine (Kleptose®, Roquette Frères, Lestrem, France), 400g d'acide citrique CAS 77-92-9, Sigma Aldrich, Saint Quentin Fallavier, France), et 120g d'hypophosphite de sodium (Sigma Aldrich, Saint Quentin Fallavier, France) sont dissous dans 2 litres d'une solution aqueuse (eau distillée). L'eau de cette solution aqueuse est ensuite évaporée à sec sous vide à 60°C sous 58 mm de mercure dans un évaporateur rotatif pendant un temps suffisant pour obtenir un mélange solide. Le résidu solide d'évaporation est ensuite chauffé sous vide à 140°C pendant 120 minutes. Puis, on met en suspension le mélange solide dans 2 litres d'eau distillée. Cette suspension est filtrée sur verre fritté. On récupère le polymère insoluble dans le filtre qui est lavé abondamment avec de l'eau puis séché à température ambiante pendant une semaine (on pourrait également sécher par exemple en étuve ventilée à 60°C pendant 24h). Le filtrat comprenant le polymère soluble est dialysé sur une membrane de 6000-8000 daltons pendant 5 jours, puis la solution dialysée est concentrée à l'aide d'un évaporateur rotatif. Le filtrat purifié est congelé puis lyophilisé, à l'aide ici d'un lyophilisateur Christ modèle alpha 1-2/ LD, à une température de -63°C, sous vide à 0,06 mbars, pendant suffisamment de temps pour obtenir un solide sous forme de poudre. Le filtrat purifié peut être, de manière alternative à la lyophilisation, également atomisé, par exemple à l'aide de l'atomiseur Büchi B-290.

On obtient ainsi 71g de polymère de cyclodextrine soluble (CDs1) et 320g de polymère de cyclodextrine insoluble (Cdi1).
- Acide poly(acrylique) (PAA) : Code du produit (323667-250G) Mw=1,800 et Mw=450 000 fournis par Sigma Aldrich®
- Alginate de sodium: code du produit (180947-500G), Lot (09611DD), Sigma Aldrich®
- Pectine de apple-pectine (Poly-D-galacturonic acid methyl ester): code du produit (75282-500G), Lot(BCBG 4396V), Sigma Aldrich®
- Acide lactique : n° de cas 50-21-5, comprenant entre 85% et 90% en masse d'acide lactique par rapport au volume total de la solution, grade ACS reagent, Sigma Aldrich®.

### I- Description du mode opératoire pour le dosage de la CEI (Capacité d'Echange Ionique) du polymère de cyclodextrine (Cds1 ou Cdi1), c'est-à-dire mesure du nombre de fonctions acides carboxyliques (-COOH) en mmoles par gramme de polymère de cyclodextrine :

100 mg de polymère (CDs1 ou Cdi1) sont dissouts dans 100 mL de solution de NaCl à 0.1M puis sont dosés par la soude 0.1 M standard en présence de phénolphtaléine comme indicateur coloré. La CEI courante est de 4 mmol environ par gramme de polymère (CDs1 ou Cdi1) ; celle-ci est assez proche de celle du chitosane qui présente environ 5 mmol de fonctions amines par gramme (pour un degré de désacétylation d'environ 80%). Le phénomène de gélification est lié à la proximité des CEI du chitosan et du polymère de cyclodextrine (selon la famille A1), et est en relation avec les proportions de chitosan et de polymère de cyclodextrine mis en présence pour la formation du gel.

### II- Exemples de préparation d'hydrogel selon l'invention et comparatifs

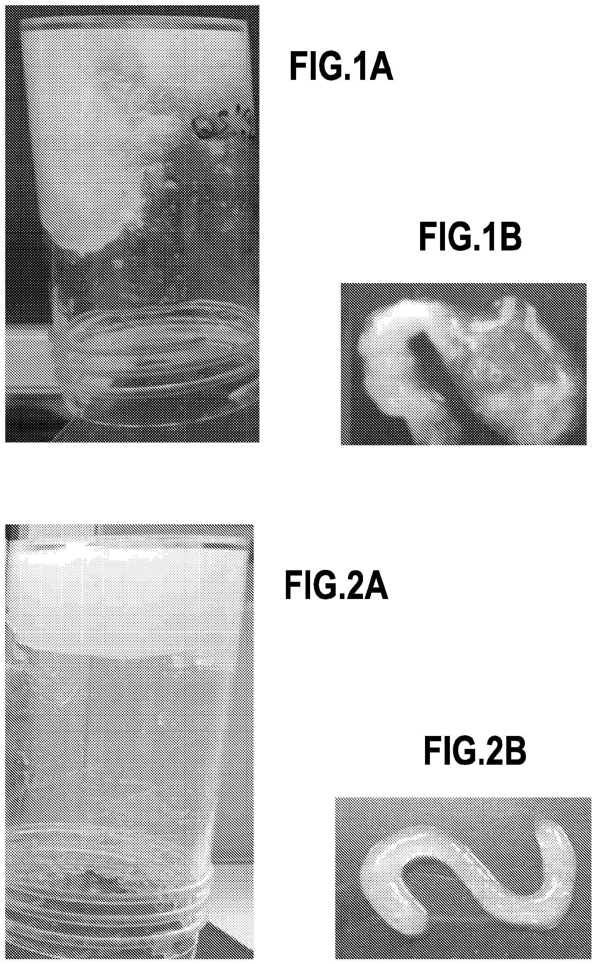

Les proportions indiquées en _{w/w} représentent le rapport entre la masse du composant ajouté et la masse totale de l'hydrogel, donc à l'issue de la quatrième étape ou de l'étape (v) du procédé selon l'invention.
Exemple 1 : 0.04g de chitosan (CHT1) (2%_{w/w}) et 0.05g (2.5%_{w/w}) du polymère de cyclodextrine (CDs1) ont été cobroyés dans un mortier pendant 1 minute. La poudre obtenue a été mise en suspension dans 1.89 g d'eau ultrapure puis mélangée au vortex pendant 15 secondes (le pH instantané de la solution est de 3.5, lequel remonte vers 4.1). 0.02 g d'une solution d'acide acétique glacial a ensuite été ajoutée à la suspension (le pH chute lors de l'ajout d'acide à 3.2 puis remonte au-dessus de 5) qui sera mélangée avec du vortex pendant 20 secondes. Un gel compact est obtenu dès les premières secondes pendant l'agitation. L'hydrogel obtenu comprend 89 % d'eau.
Exemple 2 : 0.04 g (2%_{w/w}) de chitosan (CHT1) et 0.06 g (3%_{w/w}) du polymère de cyclodextrine (CDs1) ont été cobroyés dans un mortier pendant 1 minute. La poudre obtenue a été mise en suspension dans 1.88 g d'eau ultrapure puis mélangée avec un vortex pendant 15 secondes. 0.02 g d'une solution d'acide acétique glacial a ensuite été ajoutée à la suspension qui sera mélangée au vortex pendant 20 secondes. Un gel compact est obtenu dès les premières secondes pendant l'agitation. L'hydrogel obtenu comprend 72 % d'eau.
Exemple 3 : 0.04 g (2%_{w/w}) de chitosan (CHT1) et 0.20 g (10%_{w/w}) du polymère de cyclodextrine (CDs1) ont été cobroyés dans un mortier pendant 1 minute. La poudre obtenue a été mise en suspension dans 1.74 g d'eau ultrapure puis mélangée avec un vortex pendant 15 secondes. 0.02 g d'une solution d'acide acétique glacial a ensuite été ajoutée à la suspension qui sera mélangée au vortex pendant 20 secondes. Un gel compact est obtenu dès les premières secondes pendant l'agitation. L'hydrogel obtenu comprend 60 % d'eau.
Exemple 4 : 0.05 g (2.5%_{w/w}) de chitosan (CHT1) et 0.06 g (3%_{w/w}) du polymère de cyclodextrine (CDs1) ont été cobroyés dans un mortier pendant 1 minute. La poudre obtenue a été mise en suspension dans 1.87 g d'eau ultrapure puis mélangée avec un vortex pendant 15 secondes. 0.02 g d'une solution d'acide acétique glacial a ensuite été ajoutée à la suspension qui sera mélangée au vortex pendant 20 secondes. Un gel compact est obtenu dès les premières secondes pendant l'agitation. L'hydrogel obtenu comprend 93 % d'eau.
Exemple 5 : 0.02 g (1%_{w/w}) de chitosan (CHT1) et 0.04 g (2%_{w/w}) du polymère de cyclodextrine (CDs1) ont été cobroyés dans un mortier pendant 1 minute. La poudre obtenue a été mise en suspension dans 1.92 g d'eau ultrapure puis mélangée avec un vortex pendant 15 secondes. 0.02 g d'une solution d'acide acétique à 1% a ensuite été ajouté à la suspension qui sera mélangée au vortex pendant 20 secondes. Le gel obtenu n'est pas compact et à tendance à s'écouler lentement..
Exemple 6 : 0.06 g (3%_{w/w}) de chitosane (CHT1) et 0.04 g (2%_{w/w}) du copolymère de cyclodextrine (CDs1) ont été cobroyés dans un mortier pendant 1 minute. La poudre obtenue a été mise en suspension dans 1.88 g d'eau ultrapure puis mélangée avec un vortex pendant 15 secondes. 0.02 g d'une solution d'acide acétique à 1% a ensuite été ajouté à la suspension qui sera mélangée au vortex pendant 20 secondes. Le gel obtenu dès les premières secondes pendant l'agitation est très compact, ne s'écoule pas, et apte à être moulé.
Exemple 7 : 0.04 g (2 %_{w/w}) de chitosan (CHT1) et 0.02 g (1%_{w/w}) du polymère de cyclodextrine (CDs1) ont été cobroyés dans un mortier pendant 1 minute. La poudre obtenue a été mise en suspension dans 1.92 g d'eau ultrapure puis mélangée au vortex pendant 15 secondes. 0.02 g d'une solution d'acide acétique à 1% a ensuite été ajoutée à la suspension qui sera mélangée au vortex pendant 20 secondes. Le gel obtenu a tendance à s'écouler.
Exemple 8 : 0.06g de chitosan (CHT1) (3%_{w/w}) et 0.06g (3%_{w/w}) du polymère de cyclodextrine (CDs1) ont été cobroyés dans un mortier pendant 1 minute. La poudre obtenue a été mise en suspension dans 1.86 g d'eau ultrapure puis mélangée avec un vortex pendant 15 secondes. 0.02 g d'une solution d'acide acétique glacial a ensuite été ajoutée à la suspension qui sera mélangée au vortex pendant 20 secondes. Un gel compact est formé dès les premières secondes pendant l'agitation, homogène, et ne s'écoulant pas par gravité est obtenu.
Exemple 9 : 0.06g de chitosan (CHT1) (3%_{w/w}) et 0.1g (5%_{w/w}) du polymère de cyclodextrine (CDs1) ont été cobroyés dans un mortier pendant 1 minute. La poudre obtenue a été mise en suspension dans 1.82 g d'eau ultrapure puis mélangée au vortex pendant 15 secondes. 0.02 g d'une solution d'acide acétique glacial a ensuite été ajoutée à la suspension qui sera mélangée au vortex pendant 20 secondes. Un gel compact est formé dès les premières secondes pendant l'agitation, homogène, et ne s'écoulant pas par gravité est obtenu.
Exemple 10 : identique à l'exemple 8 à la différence que le chitosan est celui de la référence CHT2. Exemple 11 : identique à l'exemple 8 à la différence que le chitosan est celui de la référence CHT3. Pour les exemples 10 et 11, l'hydrogel obtenu est ferme, compact, et homogène. Il ne s'écoule pas si on retourne le contenant.
Exemple 12 : identique à l'exemple 8 à la différence que la quantité de chitosan est de 3%_{w/w} et la quantité du polymère de cyclodextrine (CDs1) est de 10%_{w/w}. L'hydrogel obtenu est ferme et compact mais plus visqueux, épais que les autres hydrogels exemplifiés selon l'invention.
Exemple 13 comparatif: identique à l'exemple 8 à la différence que le polymère de cyclodextrine (CDs1) est remplacé par un monomère de sel de sodium de carboxyméthylβcyclodextrine commerciale (Aldrich, référence 21906). L'hydrogel obtenu s'écoule si on retourne le contenant et n'est pas ferme. Exemple 14 : identique à l'exemple 8 à la différence que le polymère de cyclodextrine (CDs1) est remplacé par un polymère d'acide polyacrylique de Mw de 1800 (ci-dessus référencé).
Exemple 14A : identique à l'exemple 8 à la différence que le polymère de cyclodextrine (CDs1) est remplacé par de la pectine (ci-dessus référencée).
Exemple 14B : identique à l'exemple 8 à la différence que le polymère de cyclodextrine (CDs1) est remplacé par de l'alginate de sodium (ci-dessus référencé).

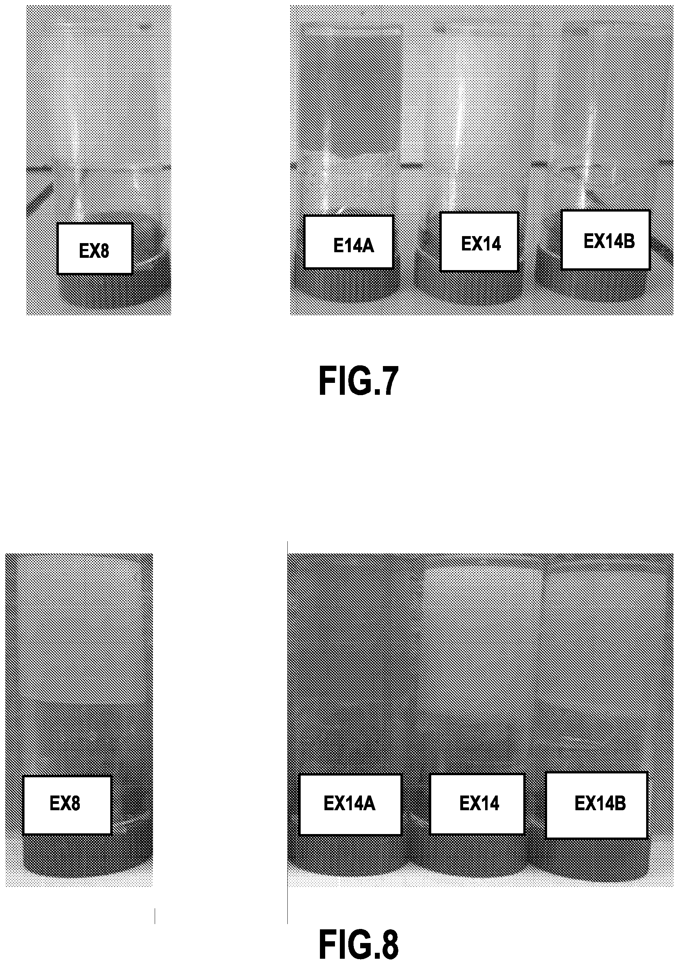

   La figure 7 représente les hydrogels des exemples 8, 14, 14A et 14B, une heure après leur formation.
   La figure 8 représente les hydrogels des exemples 8, 14, 14A et 14B après 24heures. On observe que les hydrogels formés à partir de ces polyanions sont fermes et ne s'écoulent pas même 24 heures après leur formation.
Exemple 15 : identique à l'exemple 8 à la différence que le polymère de chitosane est remplacé par un polymère d'acide polyacrylique de haut poids moléculaire (Mw : 450 000, référencé ci-dessus). Les hydrogels dans les exemples 14 et 15 obtenus sont fermes et ne s'écoulent pas.
Exemple 16 témoin : identique à l'exemple 8 à la différence que le polymère de cyclodextrine utilisé est synthétisé en remplaçant la β-cyclodextrine par la maltodextrine (d'indice d'équivalent à la dextrose D19 (19 unités de répétition), qui est commercialisé sous la marque Glucidex® D19 par Roquette), dont la structure moléculaire linéaire ne forme pas de cavité). Le gel formé est ferme et ne s'écoule pas.
Exemple 17 comparatif: 0.3 g de chitosan (CHT1) (3%_{w/w}), broyé et tamisé sur un tamis ayant des orifices de 125 µm, est solubilisé dans un volume de 9.3 mL d'eau ultrapure (93%_{w/w}) dans lequel 0.1 mL d'acide acétique glaciale est ajouté (1%_{w/w}), l'ensemble est mélangé à environ 10 000 tours/min à l'aide d'un homogénéisateur de type ultra turrax® pendant une minute. Puis, 0.3 g du polymère de cyclodextrine (CDs1(3%w/w), également broyé et tamisé sur un tamis ayant des orifices de 125 µm, est ajouté à la solution acide de chitosane (CHT1), la solution est alors mélangée à environ 10 000 tours/min à l'aide d'un homogénéisateur de type ultra-turrax® pendant 1 minute. Le temps de gel mesuré issu des valeurs de G' et G" est supérieur à 5 jours. Le gel formé est « liquide », présente des grumeaux, n'est pas homogène et n'est pas apte à être moulé (il s'écoule par action de la gravité). L'ajout du polymère anionique (CDs1) à une solution acide de chitosan ne permet donc pas de former un hydrogel de forme propre, ferme et compact. L'hydrogel obtenu est représenté sur les figures 1A et 1B. On remarque que dans le pot dans lequel il a été synthétisé à la figure 1A, l'hydrogel coule sur les parois, il n'est donc pas ferme. A la figure 1B, l'hydrogel ne conserve pas la forme de cordon qui lui a été impartie lors de sa dépose sur une surface horizontale à l'aide d'une seringue.
Exemple 18 comparatif : 0.3 g de poudre de chitosan (CHT1) (3%w/w), non tamisée et non broyée, et 0.3 g de poudre du polymère de cyclodextrine (CDs1) (3%w/w), non tamisée et non broyée, sont co-broyées à sec ce qui permet de réduire leur granulométrie et de les mélanger. Le mélange de poudres cobroyées est ajouté dans 9.3 mL d'eau ultrapure (93%_{w/w}), la solution aqueuse obtenue est alors mélangée avec du vortex pendant 20 secondes, puis 0.1 ml (1%_{v/v} d'une solution d'acide acétique glacial sont ajoutés. Le temps de gel mesuré issu des valeurs de G' et G" est supérieur à 2h et demi. La fermeté de l'hydrogel est correcte mais il présente des grumeaux.
Exemple 19 comparatif : 0.3 g de poudre de chitosan (CHT1) (3%_{w/w}), broyée et tamisée sur un tamis ayant des orifices de 125 µm, et 0.3 g de poudre du copolymère de cyclodextrine (CDs1) (3%_{w/w}), broyée et tamisée sur un tamis ayant des orifices de 125 µm, sont co-broyées à sec. Le mélange de poudres cobroyées est mis en suspension dans 9.3 ml d'eau ultrapure (93%_{w/w}) sous agitation à environ 10 000 tours/min à l'aide d'un homogénéisateur de type ultra-turrax® pendant 20 secondes. Puis, 0.1 ml (1%_{v/v}) d'une solution d'acide acétique glacial a ensuite été ajouté à la suspension qui sera mélangée sous agitation à environ 10 000 tours/min à l'aide d'un ultra-turrax® pendant 20 secondes. Le temps de gel mesuré issu des valeurs de G' et G" est supérieur à 2h30. Le gel formé a une bonne texture, est lisse, homogène et ferme, c'est-à-dire qu'il ne s'écoule pas sous l'action de la gravité et sera apte à être moulé selon une forme déterminée. Cet hydrogel est représenté aux figures 2A et 2B. On remarque à la figure 2A que l'hydrogel ne coule pas sur les parois du pot dans lequel il a été fabriqué, après son retournement et qu'il est bien ferme. A la figure 2B, on remarque que l'hydrogel conserve sa forme de cordon qui lui a été impartie en sortie d'une seringue. On remarque également qu'il est homogène, lisse et ne s'écoule pas.

Les temps de gels mesurés pour les exemples 17, 18 et 19 sont issus des courbes des modules élastiques et visqueux G' et G" établies en fonction du temps, avec une contrainte de 100%, une amplitude gamma de 100%, une fréquence angulaire oméga de 10s⁻¹ à une température de 25°C à l'aide d'un spectromètre mécanique dynamique, tels que ceux commercialisés par Anton Paar. Exemple 20 : 0.3 g de chitosan (CHT1) (3%_{w/w}), 0.3 g (3%_{w/w}) du polymère de cyclodextrine (CDs1) et 0.3 g d'hydroxyapatite (3 %_{w/w}), soit 33.3% du poids total du mélange sec de poudres, ont été cobroyés dans un mortier pendant 1 minute. Le mélange de poudres obtenu a été mis en suspension dans 9 ml d'eau ultrapure puis mélangé avec un vortex pendant 15 secondes. 0.01 ml (0,1%_{w/w}) d'une solution d'acide acétique glacial a ensuite été ajouté à la suspension qui sera mélangée avec du vortex pendant 45 secondes. Un gel compact, homogène, et ne s'écoulant pas sous l'effet de la gravité est obtenu.

### Exemples avec un milieu agueux comprenant du glucose

Exemple 27 : 0.3 g de chitosan (CHT1) (3%w/w), 0.3 g (3%w/w) du polymère de cyclodextrine (CDs1) et 0.3 g d'hydroxyapatite (3 %_{w/w}), soit 33.3% du poids total du mélange sec de poudres, ont été cobroyés dans un mortier pendant 1 minute. Le mélange de poudres obtenu a été mis en suspension dans 9 ml d'un milieu aqueux comprenant 5% en masse de glucose par rapport audit volume dudit milieux aqueux puis mélangé avec un vortex pendant 15 secondes. 0.1ml d'une solution d'acide lactique concentrée entre 85% et 90% a ensuite été ajouté à la suspension qui sera mélangée avec du vortex pendant 45 secondes. Le gel est injecté sous forme de cordon à l'aide d'une seringue dans un milieu DMEM (Dulbecco's Modified Eagle Médium) et dans un milieu type PBS. Le cordon formé est ferme (peut être manipulé à l'aide d'une spatule) et stable (jusqu'à une heure après sa formation). Exemple 28 : il s'agit du même exemple que l'exemple 27 à la différence que le mélange des poudres est effectué avec le milieux aqueux comprenant 5% en masse de glucose par rapport au volume dudit milieu, qui est déjà acidifié avec l'acide lactique, à l'aide d'un dispositif comprenant une première seringue recevant le mélange des poudres et une seconde seringue comprenant ledit volume du milieux aqueux acidifié comprenant du glucose et un organe de mise en liaison fluidique desdites première et seconde seringues. Lesdites première et seconde seringues sont en liaison fluidique permettant ainsi de faire passer le milieu aqueux acidifié dans le mélange des poudres et inversement. Cette disposition génère du cisaillement lors du mélange des poudres et du milieu aqueux favorisant ainsi la formation d'un hydrogel. Le gel formé est ensuite injecté sous forme de cordon à l'aide dudit dispositif (en ne conservant que la première ou seconde seringue) dans un milieu DMEM et dans un milieu physiologique type PBS. La fermeté et la stabilité du cordon sont améliorées comparativement à l'exemple 27.

Exemple 29 : il s'agit du même exemple que l'exemple 28 à la différence que l'acide lactique est remplacé par de l'acide chlorhydrique (0,036M). Le gel formé est ensuite injecté sous forme de cordon à l'aide dudit dispositif dans un milieu DMEM et dans un milieu physiologique type PBS. La fermeté et la stabilité du cordon sont améliorées comparativement à l'exemple 27 mais sont inférieures à celles obtenues pour l'exemple 28.

### III- Fabrication d'un matériau alvéolaire selon l'invention

Exemple 21 : L'hydrogel de l'exemple 8 a subi une étape de lyophilisation pour la formation d'un matériau alvéolaire spongieux, telle qu'une mousse, à l'aide d'un lyophilisateur Christ modèle alpha 1-2/ LD, à une température de -63°C, sous vide à 0,06 mbars, pendant au moins 24 heures. Le taux de gonflement est de 1739.0 % dans l'eau à température ambiante au bout de 6 heures et de 445.0% dans le PBS (Phosphate Buffer Saline, pH de 7.4, 0.1M) à température ambiante au bout de 6 heures ([(masse humide - masse sèche)/masse sèche]^{∗}100).

Exemple 22 : l'hydrogel de l'exemple 9 a subi une étape de lyophilisation pour la formation d'un matériau alvéolaire à l'aide d'un lyophilisateur Christ modèle alpha 1-2/ LD, à une température de - 63°C, sous vide à 0,06 mbars, pendant au moins 24 heures. Le taux de gonflement est de 262.0 % dans l'eau à température ambiante au bout de 6 heures et de 336.0% dans le PBS (Phosphate Buffer Saline, pH de e 7.4, 0.3M) à température ambiante au bout de 6 heures ([(masse humide - masse sèche)/masse sèche]^{∗}100).

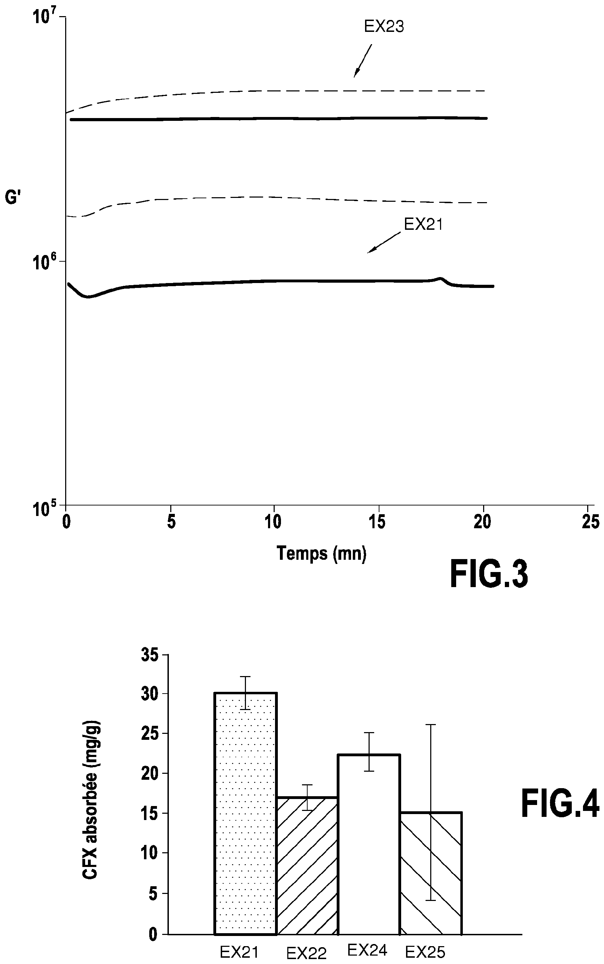

Exemple 23 : le matériau alvéolaire de l'exemple 21 subit un traitement thermique consistant à placer ce dernier dans une enceinte à 140°C pendant 1 heure. Ce traitement thermique permet de réticuler le matériau alvéolaire en transformant les liaisons ioniques en liaisons covalentes en particulier en liaisons amides. On observe alors que la résistance à la compression, c'est-à-dire à l'écrasement, du matériau alvéolaire est très significativement améliorée. Ceci s'observe visuellement en écrasant le matériau alvéolaire de l'exemple 23 entre deux doigts, ce dernier recouvre sa forme après suppression de la déformation quasi-instantanément contrairement au matériau alvéolaire des exemples 21 ou 22. On remarque à la figure 3 représentant les courbes des modules élastiques G' des exemples 22 et 21 que le module élastique G' de l'exemple 23 ayant subi un traitement thermique est nettement plus élevé que le module élastique G' de l'exemple 21.

Exemple 24 : identique à l'exemple 21 à la différence que l'hydrogel a été synthétisé avec 10% (w/w) du polymère de cyclodextrine (CDs1).

Exemple 25 témoin: l'hydrogel de l'exemple 16 a subi une étape de lyophilisation pour la formation d'un matériau alvéolaire.

Exemple 26: l'hydrogel de l'exemple 20 a subi une étape de lyophilisation à l'aide d'un lyophilisateur Christ modèle alpha 1-2/ LD, à une température de -63°C, sous vide à 0,06 mbars, pendant au moins 24 heures pour la formation d'un matériau alvéolaire contenant de l'hydroxyapatite (33.3%w/w).

### IV- Mesure de la cvtocompatibilité

La cytocompatibilité de l'hydrogel obtenue à l'exemple 8et des matériaux alvéolaires des exemples 21 et 22 a été testé selon la norme ISO 10993-5 :2009 - Évaluation biologique des dispositifs médicaux -- Partie 5: Essais concernant la cytotoxicité in vitro. La méthode de viabilité cellulaire est effectuée par la méthode du bleu alamar- test d'extraction - cellules L132 (ATCC-CCL5).

La viabilité mesurée pour l'exemple 8 est de 80% après 1 heure et est de 84% après un rinçage pendant 1 heure dans le PBS (Phosphate Buffer Saline, pH7.4, 0.1M). La viabilité mesurée pour l'exemple 21 est de 91% et est de 85% pour l'exemple 24 au bout d'une heure.

La cytotoxicité a été mesurée selon la même norme décrite ci-dessus avec des cellules MC3T3, le témoin est le TCPS (Tissue Culture Polystyrene). On obtient une valeur pour l'exemple 28 de l'ordre de 90% alors que cette valeur baisse en dessous de 60% (environ 55%) lorsque le milieu aqueux ne comprend pas de glucose. Avantageusement, lorsque le milieu aqueux comprend du glucose, la cytotoxicité est très nettement amélioré.

### V- Imprégnation des matériaux alvéolaires

40mg des matériaux alvéolaires des exemples 21,22, 24 et 25 sont imprégnées dans une solution aqueuse à 2mg/ml de ciprofloxacine à température ambiante sous agitation à 250 tours/min pendant 4h.

Les matériaux alvéolaires ainsi traités sont placés pendant 24 heures dans une solution de soude à 0.1N à 37°C afin d'extraire la ciprofloxacine adsorbée. Les solutions obtenues sont titrées au spectrophotomètre UV à une longueur d'onde de 271 nm afin de mesurer la quantité de ciprofloxacine absorbée (mg/g) pour chacun des matériaux alvéolaires. Les courbes d'absorption obtenues sont représentées à la figure 4. Les barres verticales sur chaque histogramme représentent les écarts-types. On remarque ainsi que l'écart type pour l'exemple 25 qui est à base de maltodextrine est supérieur par à celui mesuré pour les autres exemples à base de cyclodextrines. Ceci montre que le taux de chargement en principe actif est plus aléatoire si la dextrine utilisée ne présente pas de cavité hydrophobe, et ne forme donc pas de complexe d'inclusion. Les exemples 21, 22 et 24 indiquent que la capacité d'adsorption des hydrogels obtenus à partir de polymère de cyclodextrines varie selon les paramètres de préparation de l'hydrogel, toutefois les écarts ne sont pas significatifs.

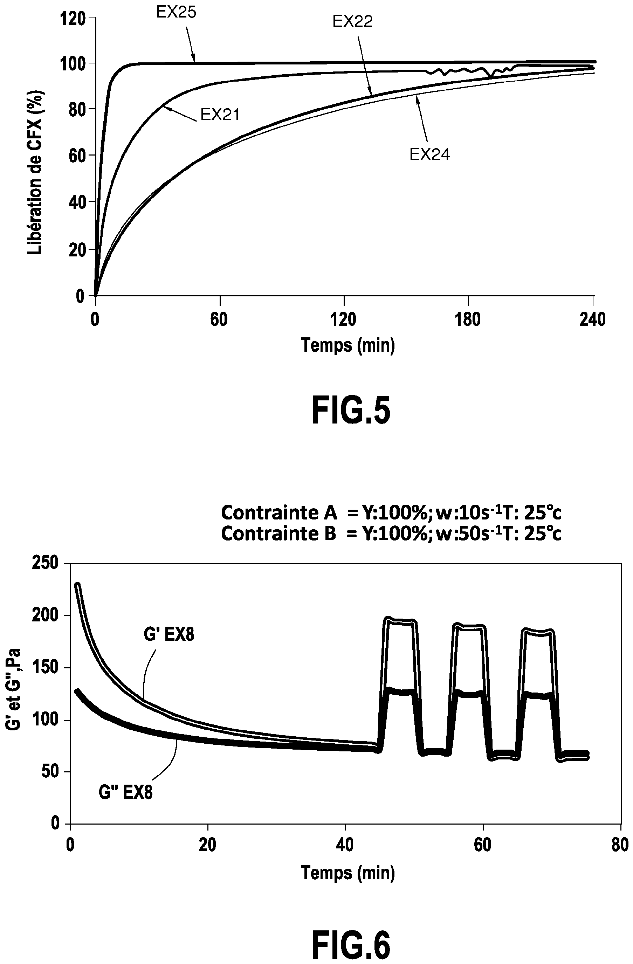

Les conditions de libération de ciprofloxacine sont étudiées en circuit fermé en injectant un flux de PBS à une température de 37°C à 30 ml/min, à partir d'un réservoir de 500 ml de PBS, dans 30 mg de matériau alvéolaire imprégné de ciprofloxacine. Le flux sortant de PBS du matériau alvéolaire est ensuite passé dans un spectrophotomètre UV (longueur d'onde de 271 nm) qui mesure la quantité de ciprofloxacine libérée, ledit flux est ensuite redirigé vers le réservoir de PBS. Le taux de libération de ciprofloxacine (%) est mesuré en rapport avec le taux absorbé mesuré précédemment et représenté à la figure 4. Les courbes des taux de libération en ciprofloxacine, représentées à la figure 5, montrent une différence très significative du profil de libération de la ciprofloxacine, qui est très rapide pour le polymère à base de maltodextrine (exemple 25), et ralenti pour les matériaux alvéolaires des exemples 21,22 et 24.

### VI- Propriétés rhéofluidifiantes de l'hydrogel selon l'invention (exemple 8)

Spécifications de l'appareil de mesure utilisé : Rhéomètre (MCR301-Anton-Paar) :

**Tableau 1**

| | |
|---|---|
| Application | Rheoplus/32 V3.00 21003114-33024 |
| Dispositif | MCR301 SN80167488 ; FW 3.11 ; Slot7 |
| Système de mesurèrent | PP25-SN17009; Gap[d=1 mm] |
| Taux de cisaillement | 1,308 min/s |
| Contrainte de cisaillement | 327,039 Pa/mNm |
| Factor de correction du moteur | 1 |
| Réglage du temps | 1 points par min |
| Amplitude gamma (γ) | 100%(premier et deuxième cycle) |
| Fréquence angulaire (ω) : | 10 s⁻¹ (premier cycle) ; 50 s⁻¹ (deuxième cycle) |
| Température | 25°C |

Le graphique représenté à la figure 6 représente l'évolution des modules élastique et visqueux G' et G" de l'hydrogel de l'exemple 8 soumis à deux cycles de contrainte A et B ; à t0, G' (230 Pa)> G" (130 Pa) puis la contrainte A appliquée pendant 45 minutes provoque une diminution de G' jusqu'à ce que sa valeur rejoigne celle de G". L'arrêt de la contrainte A suivi de l'application immédiate de la contrainte B provoque une augmentation instantanée de G' qui retrouve une valeur proche de celle du début de l'expérience (200 Pa). Après 5 minutes de contrainte B, on réapplique la contrainte A, et G' retrouve sa valeur minimale. L'alternance de contraintes A et B sur des durées de 5 minutes lors de 3 cycles successifs montre une variation réversible du module G', ce qui indique que l'hydrogel présente un comportement de type visqueux ou élastique en fonction de la contrainte appliquée. Cette caractéristique est celle d'un matériau qui présente un comportement rhéofluidifiant (et donc thixotropique). Par le biais de cette mesure, on montre que l'hydrogel présente les propriétés nécessaires pour être considéré comme injectable, c'est-à-dire applicable au moyen d'une seringue : lors de la poussée sur le piston de la seringue, le gel se fluidifie dans l'aiguille ou le cathéter par l'action des forces de cisaillement, et une fois en place dans le site de son implantation (défet osseux par exemple), l'hydrogel se fige à nouveau et retrouve une cohésion suffisante pour rester en place.

### VII- Analyse Mécanique Thermo Dynamique (DMTA) du matériau alvéolaire des exemples 21 et 23

Spécifications de l'appareil : Rhéomètre (MCR301-Anton-Paar).

**Tableau 2**

| | |
|---|---|
| Application | Rheoplus/32 V3.00 21003114-33024 |
| Dispositif | MCR301 SN80167488 ; FW 3.11 ; Slot3 ; Adj27d |
| Système de mesurèrent | RF10-SN11542 |
| Taux de cisaillement | 9,1 E-03 min/s |
| Contrainte de cisaillement | 142 725,84 Pa/mNm |
| Factor de correction du moteur | 1 |
| Angle de déflection | 11,5 mrad |
| Réglage du temps | 2points par min |
| Amplitude gamma | 0.1% |
| Fréquence : | 1Hz |
| Force normale | -1N |
| Température | 25°C |

### Caractéristiques de l'échantillon analysé par DMTA et résultats de la DMTA:

**Tableau 3**

| Echantillon | épaisseur (mm) | Longueur (mm) | Hauteur (mm) | G'(Pa) | G" (Pa) |
|---|---|---|---|---|---|
| exemple 21 | 2.49 | 7 | 29 | 1.75E+06 | 1.84E+05 |
| exemple 23 | 1.7 | 7 | 29 | 5.11E+06 | 3.79E+05 |

Le tableau 3 ci-dessus indique les résultats viscoélastiques des matériaux alvéolaires (telles des éponges) des exemples 21 et 23. Après 20 minutes d'analyse à 25°C, on observe de manière constante que le module élastique (G') est supérieur au module visqueux (G''), ce qui permet de mettre en évidence que les matériaux alvéolaires des exemples 21 et 23 présentent tous deux un comportement élastique. On remarque également que les valeurs des deux modules G' et G" de l'exemple 23 sont nettement plus élevés que celles de l'exemple 21. Le traitement thermique appliqué à l'exemple 23 améliore ainsi les performances mécaniques du matériau alvéolaire. On peut ainsi conclure que pendant cette étape de traitement thermique, le comportement intramoléculaire a été modifié, probablement du fait de transformations de liaisons ioniques en liaisons covalentes ce qui confère au matériau alvéolaire de l'exemple 23 un certain raidissement.

## Revendications

1. Procédé de fabrication d'un hydrogel **caractérisé en ce qu'**il comprend les étapes successives suivantes :
- une première étape (i) de fourniture d'au moins une poudre d'un polymère anionique (A) et d'au moins une poudre de chitosan (B) comprenant des fonctions amines (-NH₂) ;
- une seconde étape (ii) consistant à mélanger à sec au moins les poudres (A) et (B) de la première étape pour former un mélange de poudres ;
- une troisième étape (iii) de mise en suspension du mélange de poudres obtenu à l'issue de la seconde étape dans un milieu aqueux ayant un pH permettant de solubiliser le polymère anionique (A) sans solubiliser le chitosan (B) ;
- une quatrième étape (iv) d'ajout d'un acide à la suspension obtenue à l'issue de la troisième étape en sorte de former l'hydrogel ;
ou les troisième (iii) et quatrième (iv) étapes sont remplacées par une cinquième étape de mélange (v) comprenant le mélange d'un milieu aqueux acidifié comprenant au moins un composé (C) comprenant au moins une unité d'un hexose ou une unité dérivée d'un hexose, et/ou au moins un phosphate dudit composé (C), avec ledit mélange comprenant au moins les poudres (A) et (B) obtenu à l'issue de la second étape (ii).

2. Procédé selon la revendication 1, **caractérisé en ce que** la poudre (A) et/ou la poudre (B) à la première étape et/ou le mélange des poudres comprenant au moins les poudres (A) et (B) à la seconde étape est/sont tamisée(s) sur un tamis de maille inférieure ou égale à 500 µm, en particulier inférieure ou égale à 150 µm.

3. Procédé selon l'une ou l'autre des revendications 1 et 2, **caractérisé en ce que** le mélange de la seconde étape est effectué par co-broyage à sec au moins des poudres (A) et (B).

4. Procédé selon l'une quelconque des revendications précédentes, **caractérisé en ce que** le polymère anionique comprend des fonctions sulfates acides (-OS(=O)₂-OH) et/ou des sels dites fonctions sulfates acides, et/ou des fonctions acides sulfoniques (-S(=O)₂-OH) et/ou des sels dites fonctions acides sulfoniques, et/ou des fonctions acides phosphoriques (-O-P(=O)₂-OH) et/ou des sels desdites fonctions acides phosphoriques, et/ou des fonctions acides carboxyliques (-COOH) et/ou des sels dites fonctions acides carboxyliques.

5. Procédé selon l'une quelconque des revendications précédentes, **caractérisé en ce que** le polymère anionique (A) est un polymère de cyclodextrine obtenu par la réaction de polymérisation d'un mélange comprenant au moins une cyclodextrine et/ou au moins un dérivé de cyclodextrine et/ou au moins un complexe d'inclusion de cyclodextrine et/ou au moins un complexe d'inclusion dérivé de cyclodextrine, avec
- au moins un acide (poly)carboxylique et/ou son anhydride d'acide (poly)carboxylique correspondant (A1) ; ou
- au moins un pyrophosphate (A2); ou
- au moins l'épichlorhydrine, le polymère obtenu subissant une étape de carboxyalkylation en sorte de greffer des fonctions acides carboxyliques (A3).

6. Procédé selon l'une quelconque des revendications précédentes, **caractérisé en ce que** le milieu aqueux à l'étape (iii) ou à l'étape (v) comprend au moins 1 % en masse, de préférence au moins 4% en masse d'au moins un composé (C) comprenant au moins une unité d'un hexose ou une unité dérivé d'un hexose, et/ou d'au moins un phosphate dudit composé (C), par rapport au volume total dudit milieu aqueux.

7. Procédé selon l'une quelconque des revendications précédentes, **caractérisé en ce qu'**il comprend l'ajout d'au moins une charge minérale sous forme de poudre au mélange de poudres réalisé lors de la seconde étape, de préférence le rapport de la masse (g) de charge(s) minérale(s) par rapport à la masse totale du mélange de poudres est supérieur ou égal à 30%.

8. Procédé de fabrication selon la revendication 7, **caractérisé en ce que** ladite charge minérale est choisie dans la liste comprenant : les biocéramiques, en particulier les phosphates de calcium, tel que l'hydroxyapatite, le bétatriphosphate de calcium, le carbonate de calcium ; l'alumine (Al₂O₃), la zircone (ZrO₂), les verres, les verres ionomères, le dioxyde de titane, et un mélange de ces derniers.

9. Procédé de fabrication d'un matériau alvéolaire, **caractérisé en ce qu'**il comprend un procédé de fabrication d'un hydrogel selon l'une quelconque des revendications 1 à 8 pour l'obtention de l'hydrogel et **en ce qu'**il comprend en outre une étape de lyophilisation effectuée sur l'hydrogel obtenu à l'issue de la quatrième étape (iv) ou de la cinquième étape (v) pour la formation d'un matériau alvéolaire.

10. Procédé de fabrication d'un matériau alvéolaire selon la revendication 9, **caractérisé en ce qu'**un agent porogène est ajouté sous forme de poudre au mélange de poudres lors de la seconde étape (ii) du procédé de fabrication de l'hydrogel.

11. Procédé de fabrication selon l'une ou l'autre des revendications 9 et 10, **caractérisé en ce qu'**il comprend une étape de traitement thermique du matériau alvéolaire à une température supérieure ou égale à 100°C, pendant au moins 5 minutes.

12. Procédé de fabrication d'un matériau alvéolaire selon l'une quelconque des revendications 9 à 11, **caractérisé en ce qu'**il comprend une étape d'imprégnation du matériau alvéolaire dans une solution comprenant au moins un agent fonctionnel et/ou au moins une cellule vivante, et **en ce que** ledit agent fonctionnel est choisi dans une première liste (I) comprenant les anticoagulants, les anti-thrombogéniques, les agents anti-mitotiques, les agents anti-prolifération, les agents anti-adhésion, les agents anti-migration, les promoteurs d'adhésion cellulaire, les facteurs de croissance, les molécules antiparasitaires, les agents anti-inflammatoires, les vitamines, les hormones, les protéines, les antifongiques, les molécules antimicrobiennes, les antiseptiques, les antibiotiques, en particulier la ciprofloxacine, la nicotine, une ou plusieurs huiles essentielles, et le mélange de ces derniers ; et ladite au moins une cellule vivante est choisie dans une seconde liste (II) comprenant les cellules humaines, animales et végétales.

13. Dispositif d'absorption et de drainage et/ou de libération d'un agent fonctionnel et/ou de support de cellules vivantes, comprenant l'hydrogel obtenu par la mise en oeuvre du procédé décrit selon l'une quelconque des revendications 1 à 8 ou le matériau alvéolaire obtenu par la mise en œuvre du procédé décrit selon l'une quelconque des revendications 9 à 12 , **caractérisé en ce que** ledit dispositif est un dispositif choisi parmi : une seringue comprenant au moins un réservoir recevant un volume déterminé d'hydrogel ; un pansement apte à délivrer au moins un agent fonctionnel et/ou apte à drainer une plaie ; un patch apte à délivrer au moins un agent fonctionnel, tel que la nicotine.

14. Dispositif permettant la mise en œuvre du procédé selon l'une quelconque des revendications 1 à 8 pour la fabrication d'un hydrogel, comprenant une première seringue, ladite première seringue comprenant le mélange comprenant au moins les poudres (A) et (B) obtenu à l'issue de l'étape (ii) et une seconde seringue comprenant un volume déterminé d'un milieu aqueux acidifié comprenant au moins un composé (C) comprenant au moins une unité d'un hexose ou une unité dérivée d'un hexose, et/ou au moins un phosphate dudit composé (C), ledit dispositif comprenant un organe de mise en liaison fluidique des première et seconde seringues agencé en sorte de permettre le mélange du milieu aqueux acidifié comprenant au moins un composé (C) comprenant au moins une unité d'un hexose ou une unité dérivée d'un hexose, et/ou au moins un phosphate dudit composé (C) avec ledit mélange comprenant au moins les poudres (A) et (B).

15. Hydrogel susceptible d'être obtenu par la mise en œuvre du procédé de fabrication selon l'une quelconque des revendications 1 à 8, **caractérisé en ce qu'**il comprend :
- un polymère de cyclodextrine (A) obtenu par la réaction de polymérisation d'un mélange comprenant au moins une cyclodextrine et/ou au moins un dérivé de cyclodextrine et/ou au moins un complexe d'inclusion de cyclodextrine et/ou au moins un complexe d'inclusion dérivé de cyclodextrine, avec
- au moins un acide (poly)carboxylique et/ou son anhydride d'acide (poly)carboxylique correspondant (A1); ou
- au moins un pyrophosphate (A2) ; ou
- au moins l'épichlorhydrine, le polymère obtenu subissant une étape de carboxalkylation en sorte de greffer des fonctions acides carboxyliques (-COOH) (A3); et
- un polymère de chitosan (B) comprenant des fonctions amines (NH₂).

## Patentansprüche

1. Verfahren zur Herstellung eines Hydrogels, **dadurch gekennzeichnet, dass** es die folgenden aufeinanderfolgenden Schritte umfasst:
- einen ersten Schritt (i) des Zuführens von mindestens einem Pulver eines anionischen Polymers (A) und mindestens einem Pulver von Chitosan (B), umfassend Aminfunktionen (-NH₂),
- einen zweiten Schritt (ii), der darin besteht, mindestens die Pulver (A) und (B) des ersten Schritts trocken zu mischen, um eine Pulvermischung zu bilden,
- einen dritten Schritt (iii) des Suspendierens der am Ende des zweiten Schritts erhaltenen Pulvermischung in einem wässrigen Medium, das einen pH-Wert aufweist, der das Löslichmachen des anionischen Polymers (A) ohne Löslichmachen des Chitosans (B) ermöglicht,
- einen vierten Schritt (iv) des Hinzugebens einer Säure zu der am Ende des dritten Schritts erhaltenen Suspension, um das Hydrogel zu bilden,
oder der dritte (iii) und vierte (iv) Schritt werden durch einen fünften Schritt des Mischens (v) ersetzt, umfassend das Mischen eines sauer gemachten wässrigen Mediums, umfassend mindestens eine Verbindung (C), umfassend mindestens eine Einheit einer Hexose oder eine von einer Hexose abgeleitete Einheit, und/oder mindestens ein Phosphat der Verbindung (C), mit der Mischung, umfassend mindestens die Pulver (A) und (B), die am Ende des zweiten Schritts (ii) erhalten wird.

2. Verfahren nach Anspruch 1, **dadurch gekennzeichnet, dass** das Pulver (A) und/oder das Pulver (B) im ersten Schritt und/oder die Pulvermischung, umfassend mindestens die Pulver (A) und (B), im zweiten Schritt auf einem Sieb mit einer Maschengröße kleiner gleich 500 µm, insbesondere kleiner gleich 150 µm, durchgesiebt wird/werden.

3. Verfahren nach einem der Ansprüche 1 und 2, **dadurch gekennzeichnet, dass** die Mischung des zweiten Schritts durch trockenes gemeinsames Mahlen mindestens der Pulver (A) und (B) ausgeführt wird.

4. Verfahren nach einem der vorhergehenden Ansprüche, **dadurch gekennzeichnet, dass** das anionische Polymer saure Sulfatfunktionen (-O-S(=O)₂-OH) und/oder Salze der sauren Sulfatfunktionen, und/oder Sulfonsäurefunktionen (-S(=O)₂-OH) und/oder Salze der Sulfonsäurefunktionen, und/oder Phosphorsäurefunktionen (-O-P(=O)₂-OH) und/oder Salze der Phosphorsäurefunktionen, und/oder Carbonsäurefunktionen (-COOH) und/oder Salze der Carbonsäurefunktionen umfasst.

5. Verfahren nach einem der vorhergehenden Ansprüche, **dadurch gekennzeichnet, dass** das anionische Polymer (A) ein Cyclodextrinpolymer ist, das durch die Polymerisationsreaktion einer Mischung, umfassend mindestens ein Cyclodextrin und/oder mindestens ein Cyclodextrinderivat und/oder mindestens ein Addukt von Cyclodextrin und/oder mindestens ein von Cyclodextrin abgeleitetes Addukt, erhalten wird mit
- mindestens einer (Poly)carbonsäure und/oder ihrem entsprechenden (Poly)carbonsäureanhydrid (A1) oder
- mindestens einem Pyrophosphat (A2) oder
- mindestens dem Epichlorhydrin, dem Polymer, das unter Einfluss eines Carboxyalkylierungsschritts erhalten wird, um Carbonsäurefunktionen (A3) zu übertragen.

6. Verfahren nach einem der vorhergehenden Ansprüche, **dadurch gekennzeichnet, dass** das wässrige Medium in Schritt (iii) oder in Schritt (v) mindestens 1 Masse-%, vorzugsweise mindestens 4 Masse-% von mindestens einer Verbindung (C), umfassend mindestens eine Einheit einer Hexose oder eine von einer Hexose abgeleitete Einheit, und/oder mindestens ein Phosphat der Verbindung (C), in Bezug auf das Gesamtvolumen des wässrigen Mediums umfasst.

7. Verfahren nach einem der vorhergehenden Ansprüche, **dadurch gekennzeichnet, dass** es das Hinzugeben von mindestens einem Mineralfüllstoff in Pulverform zu der Pulvermischung umfasst, die in dem zweiten Schritt umgesetzt wurde, wobei das Verhältnis der Masse (g) des Mineralfüllstoffs bzw. der Mineralfüllstoffe in Bezug auf die Gesamtmasse der Pulvermischung vorzugsweise größer gleich 30 % ist.

8. Herstellungsverfahren nach Anspruch 7, **dadurch gekennzeichnet, dass** der Mineralfüllstoff aus der Liste ausgewählt ist, umfassend: Biokeramiken, insbesondere Calciumphosphate, wie Hydroxylapatit, Calciumbetatriphosphat, Calciumcarbonat, Aluminiumoxid (Al₂O₃), Zirkoniumoxid (ZrO₂), Gläser, Ionomergläser, Titandioxid und eine Mischung davon.

9. Herstellungsverfahren eines geschäumten Materials, **dadurch gekennzeichnet, dass** es ein Herstellungsverfahren eines Hydrogels nach einem der Ansprüche 1 bis 8 zum Erhalten des Hydrogels umfasst, und dadurch, dass es ferner einen Schritt zur Lyophilisierung umfasst, der an dem am Ende des vierten Schritts (iv) oder des fünften Schritts (v) erhaltenen Hydrogel zur Bildung eines geschäumten Materials ausgeführt wird.

10. Herstellungsverfahren eines geschäumten Materials nach Anspruch 9, **dadurch gekennzeichnet, dass** während des zweiten Schritts (ii) des Herstellungsverfahrens des Hydrogels ein Treibmittel in Pulverform zu der Pulvermischung hinzugegeben wird.

11. Herstellungsverfahren nach einem der Ansprüche 9 und 10, **dadurch gekennzeichnet, dass** es mindestens für 5 Minuten einen Schritt der Wärmebehandlung des geschäumten Materials bei einer Temperatur von größer gleich 100 °C umfasst.

12. Herstellungsverfahren eines geschäumten Materials nach einem der Ansprüche 9 bis 11, **dadurch gekennzeichnet, dass** es einen Imprägnierschritt des geschäumten Materials in einer Lösung, umfassend mindestens ein funktionales Mittel und/oder mindestens eine lebende Zelle, umfasst, und dadurch, dass das funktionale Mittel aus einer ersten Liste (I) ausgewählt ist, umfassend gerinnungshemmende Mittel, Thrombosehemmer, Antimitotika, Proliferationshemmer, Adhäsionshemmer, Migrationshemmer, Zelladhäsionspromotoren, Wachstumsfaktoren, antiparasitäre Moleküle, Entzündungshemmer, Vitamine, Hormone, Proteine, Fungizide, antimikrobielle Moleküle, Antiseptika, Antibiotika, insbesondere Ciprofloxacin, Nikotin, ein oder mehrere ätherische Öle, und die Mischung davon, und die mindestens eine lebende Zelle aus einer zweiten Liste (II) ausgewählt ist, umfassend menschliche, tierische und pflanzliche Zellen.

13. Vorrichtung zur Absorption und Drainage und/oder Freisetzung eines funktionalen Mittels und/oder zur Unterstützung von lebenden Zellen, umfassend das durch die Umsetzung des beschriebenen Verfahrens nach einem der Ansprüche 1 bis 8 erhaltene Hydrogel oder das durch die Umsetzung des beschriebenen Verfahrens nach einem der Ansprüche 9 bis 12 erhaltene geschäumte Material, **dadurch gekennzeichnet, dass** die Vorrichtung eine Vorrichtung ist, die ausgewählt ist aus: einer Spritze, umfassend mindestens ein Reservoir, das ein bestimmtes Volumen eines Hydrogels aufnimmt, einen Verband, der geeignet ist, um mindestens ein funktionales Mittel abzugeben und/oder eine Wunde zu drainieren, einem Pflaster, das dazu geeignet ist, mindestens ein funktionales Mittel, wie Nikotin, abzugeben.

14. Vorrichtung, welche die Umsetzung des Verfahrens nach einem der Ansprüche 1 bis 8 zur Herstellung eines Hydrogels ermöglicht, umfassend eine erste Spritze, wobei die erste Spritze die Mischung umfasst, die mindestens die Pulver (A) und (B) umfasst, und die am Ende von Schritt (ii) erhalten wird, und eine zweite Spritze, umfassend ein bestimmtes Volumen eines sauer gemachten wässrigen Mediums, umfassend mindestens eine Verbindung (C), umfassend mindestens eine Einheit einer Hexose oder eine von einer Hexose abgeleitete Einheit, und/oder mindestens ein Phosphat der Verbindung (C), wobei die Vorrichtung ein Organ zur Herstellung einer fluidischen Verbindung der ersten und zweiten Spritze umfasst, das angeordnet ist, um die Mischung des sauer gemachten wässrigen Mediums, umfassend mindestens eine Verbindung (C), umfassend mindestens eine Einheit einer Hexose oder eine von einer Hexose abgeleitete Einheit, und/oder mindestens ein Phosphat der Verbindung (C), mit der Mischung, umfassend mindestens die Pulver (A) und (B), zu ermöglichen.

15. Hydrogel, das dazu geeignet ist, durch die Umsetzung des Herstellungsverfahrens nach einem der Ansprüche 1 bis 8 erhalten zu werden, **dadurch gekennzeichnet, dass** es umfasst:
- ein Cyclodextrinpolymer (A), das durch die Polymerisationsreaktion einer Mischung, umfassend mindestens ein Cyclodextrin und/oder mindestens ein Cyclodextrinderivat und/oder mindestens ein Addukt von Cyclodextrin und/oder mindestens ein von Cyclodextrin abgeleitetes Addukt, erhalten wird mit
- mindestens einer (Poly)carbonsäure und/oder ihrem entsprechenden (Poly)carbonsäureanhydrid (A1) oder
- mindestens einem Pyrophosphat (A2) oder
- mindestens dem Epichlorhydrin, dem Polymer, das unter Einfluss eines Carboxyalkylierungsschritts erhalten wird, um Carbonsäurefunktionen (-COOH) (A3) zu übertragen, und
- einem Chitosanpolymer (B), umfassend Aminfunktionen (NH₂).

## Claims

1. A method of producing a hydrogel, **characterized in that** it comprises the following steps in succession:
- a first step (i) of providing at least one powder of an anionic polymer (A) and at least one chitosan powder (B) comprising amine functions (-NH₂);
- a second step (ii) consisting in dry mixing at least the powders (A) and (B) from the first step in order to form a mixture of powders;
- a third step (iii) of suspending the mixture of powders obtained from the second step in an aqueous medium having a pH that can enable the anionic polymer (A) to be dissolved without dissolving the chitosan (B);
- a fourth step (iv) of adding an acid to the suspension obtained from the third step in order to form the hydrogel; or
the third (iii) and fourth (iv) steps are replaced by a mixing fifth step (v), comprising mixing an acidified aqueous medium including at least one compound (C) comprising at least one unit of a hexose or a unit derived from a hexose, and/or at least one phosphate of said compound (C), with said mixture comprising at least the powders (A) and (B) obtained from the second step (ii) .

2. A method according to claim 1, **characterized in that** the powder (A) and/or the powder (B) in the first step and/or the mixture of powders comprising at least the powders (A) and (B) in the second step is/are sieved over a sieve with a mesh less than or equal to 500 µm, in particular less than or equal to 150 µm.

3. A method according to claim 1 or claim 2, **characterized in that** the mixing of the second step is carried out by dry co-milling at least the powders (A) and (B).

4. A method according to any preceding claim, **characterized in that** the anionic polymer comprises acid sulfate functions (-O-S(=O)₂-OH or -SO₄H) and/or salts of said acid sulfate functions, and/or sulfonic acid functions (-S(=O)₂-OH or -SO₃H) and/or salts of said sulfonic acid functions, and/or phosphoric acid functions (-O-P(=O)₂-OH or -PO₄H) and/or salts of said phosphoric acid functions, and/or carboxylic acid functions (-COOH) and/or salts of said carboxylic acid functions.

5. A method according to any preceding claim, **characterized in that** the anionic polymer (A) is a cyclodextrin polymer obtained by a reaction for polymerization of a mixture comprising at least one cyclodextrin and/or at least one cyclodextrin derivative and/or at least one inclusion complex of cyclodextrin and/or at least one inclusion complex of a cyclodextrin derivative, with
- at least one (poly)carboxylic acid and/or its corresponding (poly)carboxylic acid anhydride (A1); or
- at least one pyrophosphate (A2); or
- at least epichlorhydrin, the polymer obtained undergoing a step for carboxyalkylation in a manner such as to graft carboxylic acid functions (A3).

6. A method according to any preceding claim, **characterized in that** the aqueous medium in step (iii) or in step (v) comprises at least 1% by weight, preferably at least 4% by weight, of at least one compound (C) comprising at least one unit of a hexose or a unit derived from a hexose, and/or of at least one phosphate of said compound (C), relative to the total volume of said aqueous medium.

7. A method according to any preceding claim, **characterized in that** it comprises adding at least one mineral filler in the form of a powder to the mixture of powders produced during the second step, with the ratio of the weight (g) of mineral filler(s) relative to the total weight of the mixture of powders preferably being 30% or more.

8. A method according to claim 7, **characterized in that** said mineral filler is selected from the list comprising: bioceramics, in particular phosphates of calcium such as hydroxyapatite, calcium betatriphosphate, calcium carbonate; alumina (Al₂O₃), zirconia (ZrO₂), glasses, glass ionomer, titanium dioxide, and mixtures thereof.

9. A method of producing a cellular material, **characterized in that** it comprises a method of producing a hydrogel according to any one of claims 1 to 8 in order to obtain the hydrogel and **in that** it furthermore comprises a step for freeze drying carried out on the hydrogel obtained from the fourth step (iv) or the fifth step (v) in order to form a cellular material.

10. A method of producing a cellular material according to claim 9, **characterized in that** a pore-forming agent is added in the form of a powder to the mixture of powders during the second step (ii) of the method of producing the hydrogel.

11. A method of producing a cellular material according to claim 9 or claim 10, **characterized in that** it includes a heat treatment step of treating the cellular material at a temperature greater than or equal to 100°C, for at least 5 minutes.

12. A method of producing a cellular material according to any one of claims 9 to 11, **characterized in that** it includes a step of impregnating cellular material in a solution comprising at least one functional agent and/or at least one living cell, and **in that** said functional agent is selected from a first list (I) comprising anticoagulants, anti-thrombogenics, antimitotics, anti-proliferation agents, anti-adhesion agents, anti-migration agents, cellular adhesion promoters, growth factors, antiparasitic molecules, anti-inflammatory agents, vitamins, hormones, proteins, antifungals, antimicrobial molecules, antiseptics, antibiotics, in particular ciprofloxacin, nicotine, one or more essential oils, and a mixture thereof; and said at least one living cell is selected from a second list (II) comprising human, animal and plant cells.

13. A device for absorbing and draining and/or for releasing a functional agent and/or for supporting living cells, comprising the hydrogel obtained by carrying out the method described in any one of claims 1 to 8 or the cellular material obtained by carrying out the method described in any one of claims 9 to 12, **characterized in that** said device is a device selected from: a syringe comprising at least one reservoir receiving a predetermined volume of hydrogel; a dressing which is capable of delivering at least one functional agent and/or capable of draining a wound; a patch that is capable of delivering at least one functional agent such as nicotine.

14. A device for carrying out the method according to any one of claims 1 to 8 for the production of a hydrogel, comprising a first syringe, said first syringe comprising the mixture comprising at least the powders (A) and (B) obtained from the step (ii) and a second syringe comprising a predetermined volume of an acidified aqueous medium comprising at least one compound (C) comprising at least one unit of a hexose or a unit derived from a hexose, and/or at least one phosphate of said compound (C), said device comprising a means for placing the first and second syringe in fluid communication, in a manner such as to allow the acidified aqueous mixture comprising at least one compound (C) comprising at least one unit of a hexose or a unit derived from a hexose and/or at least one phosphate of said compound (C) to be combined with said mixture comprising at least the powders (A) and (B).

15. A hydrogel that is capable of being obtained by carrying out the production method according to any one of claims 1 to 8, **characterized in that** it comprises:
- a cyclodextrin polymer (A) obtained by a reaction for polymerization of a mixture comprising at least one cyclodextrin and/or at least one cyclodextrin derivative and/or at least one inclusion complex of cyclodextrin and/or at least one inclusion complex of a cyclodextrin derivative, with
- at least one (poly)carboxylic acid and/or its corresponding (poly)carboxylic acid anhydride (A1); or
- at least one pyrophosphate (A2); or
- at least epichlorhydrin, the polymer obtained undergoing a step for carboxyalkylation in a manner such as to graft carboxylic acid functions (-COOH) (A3); and
- a chitosan polymer (B) comprising amine functions (NH₂).
